# EUROPÄISCHE PATENTSCHRIFT

(11) **EP 4 178 650 B1**
(45) Veröffentlichungstag und Bekanntmachung des Hinweises auf die Patenterteilung: **08.05.2024**
(21) Anmeldenummer: 21745931.2
(22) Anmeldetag: 06.07.2021
(51) Int. Cl.: A61M 16/00, F04D 25/08, F04D 29/42, F04D 29/58, H02K 7/14, H02K 5/02, H02K 9/22

(54) **NOTFALL-BEATMUNGSGERÄT MIT KONDUKTIV UND KONVEKTIV GEKÜHLTEM GEBLÄSE**
EMERGENCY VENTILATOR WITH A FAN WHICH IS COOLED IN A CONDUCTIVE AND CONVECTIVE MANNER
RESPIRATEUR D'URGENCE DOTÉ D'UN VENTILATEUR REFROIDI PAR CONDUCTION ET PAR CONVECTION

(30) Priorität: 13.07.2020 DE 102020118466
(43) Veröffentlichungstag der Anmeldung: 17.05.2023
(73) Patentinhaber: Hamilton Medical AG, 7402 Bonaduz (CH)
(72) Erfinder: HEPTING, Daniel, 7026 Maladers (CH); HUNGER, Jan, 7000 Chur (CH)
(74) Vertreter: Ruttensperger Lachnit Trossin Gomoll
(86) Internationale Anmeldenummer: PCT/EP2021/068636
(87) Internationale Veröffentlichungsnummer: WO 2022/013006

(56) Entgegenhaltungen:
- EP-A1- 3 391 924
- WO-A1-2008/098382
- US-A- 4 794 922
- US-A1- 2013 263 854
- US-A1- 2019 366 025

## Beschreibung

Die vorliegende Erfindung betrifft ein Notfall-Beatmungsgerät gemäß dem Oberbegriff von Anspruch 1.

Ein gattungsgemäßes Notfallgerät ist aus der US 2019/0366025 A1 bekannt.

Außerdem ist aus der US 2013/0263854 A1 ein tragbares Beatmungsgerät mit einem pneumatischen Blockmodul bekannt, wobei das pneumatische Blockmodul umfasst: eine Spiralbaugruppe, die einen Lufteinlasskanal, eine Halterung für ein Gebläse und einen Luftauslasskanal umfasst; wobei das Gebläse in der Halterung so angebracht ist, dass ein Flügelrad des Gebläses in einem Strömungskanal liegt, der den Lufteinlasskanal und den Luftauslasskanal verbindet; ein Gehäuse, das die Spiralbaugruppe umschließt, wobei Luftkanäle innerhalb des Gehäuses Luftanschlüsse an der Spiralbaugruppe verbinden. Der Lufteinlasskanal der Spiralbaugruppe steht in Fluidverbindung mit einem Umgebungslufteinlass des Beatmungsgeräts. Der Luftauslasskanal der Spiralbaugruppe steht in Fluidverbindung mit dem Luftauslass des Beatmungsgeräts.

Die US 4794922 offenbart ein Beatmungsgerät für die künstliche Beatmung eines Patienten mit einer Vielzahl von pneumatischen Komponenten, die auf einem Verteiler montiert sind. Der Verteiler ist ein einteiliges Aluminiumgussteil mit einer Basis, in der Kanäle ausgebildet sind, die eine Fluidverbindung zwischen den pneumatischen Komponenten ermöglichen. Die Kanäle liegen an der Unterseite des Sockels frei und sind gegenüber der Atmosphäre durch eine Dichtung und eine Dichtungsplatte abgedichtet, die an der Unterseite des Sockels so befestigt sind, dass sie den freiliegenden Teil der Kanäle abdecken. Im Verteiler sind mehrere Komponenten als Teil des Gussteils geformt, darunter ein Akkumulator und ein Pulsationsdämpfer, die beide Gasspeicherkammern sind. Mehrere Rippen sind ebenfalls als Teil des Verteilers ausgebildet und dienen als Wärmesenke für eine Leiterplatte, die am Verteiler befestigt ist. Abnehmbare pneumatische Komponenten sind an Montageblöcken befestigt, die sich von der Basis nach oben erstrecken und mit Gewindebohrungen für die Aufnahme von Befestigungselementen versehen sind. Die Fluidverbindung zwischen den Kanälen und den pneumatischen Komponenten wird durch Stutzen hergestellt, die sich von der Basis nach oben erstrecken und in die die pneumatischen Komponenten dichtend eingreifen. Eine Bohrung durch die Stutzen bildet eine Zugangsöffnung zu den Kanälen. Die Zugangsöffnungen sind mit den Einlässen und Auslässen der pneumatischen Komponenten ausgerichtet, wenn die pneumatischen Komponenten an den Montageblöcken befestigt sind.

Als weitere Notfall-Beatmungsgeräte sind bekannt das mobile Beatmungsgerät mit der Bezeichnung "EOVE-150 Ventilator" der Fa. EOVE SA in Pau (FR), das mobile Beatmungsgerät mit der Bezeichnung "EVE_{IN}" der Fritz Stephan GmbH in Gackenbach (DE) sowie das mobile Beatmungsgerät mit der Bezeichnung "Falco 202 Evo" der italienischen Firma Siare Engineering International Group s. r. I. in Valsamoggia (IT).

Aus der EP 3 391 924 A1 ist ein mobiles Beatmungsgerät bekannt, dessen Beatmungsgasstrom als konvektiver Kühlgasstrom an einer Wandung des Beatmungsgeräts entlang geführt wird, mit welcher ein Akkumulator als Energiespeicher des Beatmungsgeräts in Kontakt ist. Dadurch soll Wärme des Akkumulators vom Beatmungsgasstrom abgeführt werden, um den Akkumulator zu kühlen und so kürzere Ladezeiten zu ermöglichen.

Notfall-Beatmungsgeräte, unter anderem auch als "intensivmedizinische Beatmungsgeräte" bezeichnet, dienen der schnellen Versorgung eines Patienten mit Beatmungsgas außerhalb einer klinischen Umgebung, also etwa an einem Unfallort oder/und während eines Transports eines Patienten. Selbstverständlich können Notfall-Beatmungsgeräte auch in einer klinischen Umgebung eingesetzt werden, jedoch stehen in Kliniken häufig leistungsfähigere Beatmungsgeräte als Notfall-Beatmungsgeräte zur Verfügung.

Das Gebläse mit dem relativ zum Gehäuse des Notfall-Beatmungsgeräts ruhenden Gebläsegehäuse und dem im Gebläsegehäuse beweglich aufgenommenen und sich während eines Beatmungsbetriebs bewegenden Luftförderer erzeugt, wie alle Vorrichtungen mit aneinander gelagerten relativ beweglichen Bauteilen, Wärme aufgrund von Reibung sowie aufgrund von elektrischem Widerstand in einem elektrischen Antrieb des Luftförderers. Ein anderer als ein elektrischer Antrieb ist zwar grundsätzlich möglich, kommt jedoch aus praktischen Gründen nicht infrage.

Aus der WO 2008/098382 A1 der vorliegenden Anmelderin ist ein Gebläse für ein Beatmungsgerät bekannt, welches zur Minderung der Schallemission durch eine Heatpipe, selterner auch als "Wärmerohr" bezeichnet, gekühlt wird.

Es ist Aufgabe der vorliegenden Erfindung, das Gebläse als Wärmequelle des Notfall-Beatmungsgeräts vor Überhitzung zu schützen und hierzu das Gewicht des Notfall-Beatmungsgeräts möglichst nicht zu erhöhen, sodass es als portables Beatmungsgerät ohne weitere Einschränkung einsetzbar bleibt.

Diese Aufgabe löst die vorliegende Erfindung durch ein Notfall-Beatmungsgerät mit allen Merkmalen des Anspruchs 1. Bei einem solchen Notfall-Beatmungsgerät ist zusätzlich das Gebläsegehäuse unter Zwischenanordnung eines Wärmeleitkörpers an dem Gehäuse festgelegt, wobei der Wärmeleitkörper ein Material umfasst oder aus einem Material gebildet ist, welches ausgewählt ist aus Leichtmetall, Buntmetall oder/und einem mit wärmeleitfähigem Füllmaterial gefüllten Kunststoff. Somit kann das Gebläsegehäuse im Gebläse entstehende Wärme an den Wärmeleitkörper wenigstens durch Wärmeleitung, aber möglicherweise auch durch zusätzliche weitere physikalische Prinzipien des Wärmetransports, wie Strahlung und Konvektion, abgeben. Das Notfall-Beatmungsgerät kann die vom Gebläsegehäuse an den Wärmeleitkörper übertragene Wärme über sein Gehäuse an die Außenumgebung des Notfall-Beatmungsgeräts übertragen.

Da das Gebläse ohnehin im Gehäuse des Notfall-Beatmungsgeräts gehäusefest angeordnet werden muss, kann durch geeignete Materialwahl der das Gebläsegehäuse halternden Struktur im Gebläse entstehende Wärme zum Gehäuse und von dort an die Umgebung abgegeben werden. Eine zusätzliche Kühlstruktur ist somit nicht notwendig. Diese das Gebläsegehäuse halternde Struktur ist zur Sicherstellung ihrer ausreichenden Wärmeleitfähigkeit wenigstens teilweise, vorzugsweise vollständig, aus wenigstens einem der genannten Materialien gebildet. Aufgrund der Ausrichtung der Struktur nicht nur auf die Festlegung des Gebläses im Gehäuse, sondern auch auf die Abführung von Wärme aus dem Gebläse während des Gebläsebetriebs und danach, ist die das Gebläsegehäuse halternde Struktur der vorliegenden Anmeldung als "Wärmeleitkörper" bezeichnet.

"Gehäusefest" bedeutet dabei nicht notwendigerweise unmittelbar am Gehäuse ausgebildet, wenngleich dies auch von dem Begriff "gehäusefest" umfasst ist. "Gehäusefest" bedeutet "vom Gehäuse, außer zu etwaigen Reparaturzwecken, bestimmungsgemäß nicht trennbar bzw. nicht entfernbar".

Von den genannten Materialien ist das Leichtmetall bevorzugt, da dieses nicht nur eine sehr hohe Festigkeit, sondern ebenfalls eine hohe Wärmeleitfähigkeit bei verhältnismäßig geringer Dichte aufweist. Bei den meisten mit wärmeleitfähigem Material gefüllten Kunststoffen ist zwar die Dichte noch niedriger als bei Leichtmetallen, jedoch ist auch die Wärmeleitfähigkeit geringer. Buntmetalle weisen üblicherweise ebenfalls eine hervorragende Wärmeleitfähigkeit auf, jedoch in den meisten Fällen bei höherer Dichte als Leichtmetalle und gefüllte Kunststoffe.

Das Gebläse weist selbstverständlich einen Antrieb auf, welcher den Luftförderer relativ zum Gebläsegehäuse in Bewegung versetzt, um Beatmungsgas in Richtung zur Beatmungsgas-Ausgabeöffnung zu fördern. Da bei dem vorliegend diskutierten Notfall-Beatmungsgerät das dem Patienten zugeführte Beatmungsgas in der Regel Umgebungsluft enthält, wobei dieser wenigstens ein von Umgebungsluft verschiedenes Sondergas beigemischt sein kann, ist die Beatmungsgas fördernde, relativ zum Gebläsegehäuse bewegliche Formation des Gebläses als "Luftförderer" bezeichnet. Der Luftförderer ist in den meisten Fällen ein rotatorisch beweglicher Luftförderer, beispielsweise ein Flügelrad oder Turbinenrad. So kann während des Betriebs des Gebläses ein konstanter Luftstrom erzeugt werden.

Das Sondergas, in der Regel Sauerstoff oder ein Anästhetikum, wie etwa Lachgas, kann durch einen Sondergas-Kopplungsabschnitt oder/und durch einen Sondergas-Hilfseinlass in das Gehäuse eingeleitet werden. Bevorzugt weist das Notfall-Beatmungsgerät eine Mischkammer auf, in welcher unterschiedliche in das Gehäuse eingeleitete bzw. eingesaugte Gase zum Beatmungsgas vermischt werden. Der Sondergas-Kopplungsabschnitt ist zur bestimmungsgemäß lösbaren Kopplung mit einer Sauerstoffversorgung oder einem Sauerstoffvorrat ausgebildet, etwa in Form einer Schnellkupplung.

Der Antrieb des Gebläses kann außerhalb oder innerhalb des Gebläsegehäuses angeordnet sein. Bevorzugt ist der Antrieb im Gebläsegehäuse angeordnet, um einerseits den Antrieb durch das Gebläsegehäuse vor äußeren Einflüssen abschirmen zu können und um andererseits auch Wärme aus dem Antrieb über das Gebläsegehäuse an den Wärmeleitkörper übertragen zu können.

Wie eingangs ausgesagt ist, ist der Luftförderer im Gebläsegehäuse aufgenommen.

Das Gebläsegehäuse kann wenigstens zweiteilig ausgebildet sein und ein den Antrieb aufnehmendes Antriebsgehäuseteil sowie ein den Luftförderer aufnehmendes Fördergehäuseteil umfassen. Das Antriebsgehäuseteil kann zur verbesserten Wärmeleitung aus einem ersten Werkstoff, etwa Metall, insbesondere aus einer Aluminiumlegierung gebildet sein, während das Fördergehäuseteil aus einem vom ersten verschiedenen zweiten Werkstoff hergestellt sein kann. Da es für das Fördergehäuseteil ausreichen kann, den Luftförderer mechanisch gegen äußere Einflüsse abzuschirmen, kann das Fördergehäuseteil aus Kunststoff als dem zweiten Werkstoff hergestellt sein, etwa als mehrteiliges Spritzgussbauteil. Das Fördergehäuseteil kann am Antriebsgehäuseteil festgelegt sein.

Das Notfall-Beatmungsgerät weist eine Steuervorrichtung auf, welche dazu ausgebildet ist, das Gebläse nur während Inspirationsphasen zu betreiben, in welchen einem Patienten Beatmungsgas zugeführt wird. Während Exspirationsphasen wird das Gebläse dagegen nicht betrieben, was nicht nur die Wärmeentwicklung im Gebläse über die gesamte Dauer einer künstlichen Beatmung mindert, sondern auch die Nutzungsdauer eines geladenen elektrischen Energiespeichers erhöht. Der elektrische Energiespeicher, bevorzugt ein mehrfach aufladbarer Akkumulator, ist in einem Aufnahmefach im Gehäuse des Beatmungsgeräts aufgenommen. Das Aufnahmefach ist bevorzugt vom Wärmeleitkörper baulich getrennt, etwa durch eine innere Gehäusetrennwand. Zusätzlich oder alternativ ist das Aufnahmefach außerhalb des Gebläsegehäuses ausgebildet, besonders bevorzugt auch von diesem baulich getrennt. Diese bauliche Trennung kann wiederum durch die bereits genannte innere Gehäusetrennwand realisiert sein. Die innere Gehäusetrennwand ist eine Trennwand im Gehäuse des Beatmungsgeräts, nicht im Gebläsegehäuse.

Das Gebläsegehäuse ist zur Erleichterung der Montage des Gebläses bevorzugt mehrteilig ausgebildet und weist beispielsweise wenigstens zwei oder noch mehr miteinander verbundene Gehäusebauteile auf.

Bei dem eingangs genannten bekannten Gerät "EOVE-150 Ventilator" ist das Gebläsegehäuse aus Kunststoff hergestellt und schwimmend, also mit gewissen Bewegungsspiel, in dem ebenfalls aus Kunststoff hergestellten Gehäuse des Beatmungsgeräts gelagert. Somit wird eine nahezu ausschließlich konvektive Kühlung des EOVE-Geräts erreicht. Allerdings kann das gerade im Bereich des rotierenden Luftförderers vorgesehene Bewegungsspiel des EOVE-Geräts negative Einflüsse auf die Drehlagerung und den Drehbetrieb des bekannten Luftförderers haben.

Bevorzugt ist daher im Notfall-Beatmungsgerät der vorliegenden Erfindung das Gebläsegehäuse starr mit dem Wärmeleitkörper verbunden. Das Gebläsegehäuse kann mit wenigstens einem Gebläsegehäusebauteil einstückig mit dem Wärmeleitkörper ausgebildet sein, was eine besonders hohe Effizienz der Wärmeleitung ermöglicht, da keine Wärmeleitwiderstände in Form von Luftspalten und dergleichen vorhanden sind. Alternativ kann das Gebläsegehäuse stoffschlüssig mit dem Wärmeleitkörper verbunden sein, etwa durch Schweißen, Löten oder Kleben. Weiter alternativ kann das Gebläsegehäuse mit wärmeleitenden Verbindungsmitteln, wie Schrauben aus Metall oder miteinander steckbaren, komplementären Vorsprüngen und Ausnehmungen im Gebläsegehäuse einerseits und im Wärmeleitkörper andererseits, mit dem Wärmeleitkörper verbindbar oder verbunden sein.

Zwischen dem Gebläsegehäuse und einer Gebläse-Verbindungsfläche des Wärmeleitkörpers, welche dem Gebläsegehäuse zugewandt ist, kann Wärmeleitpaste oder eine Wärmeleitmatte angeordnet sein, um etwaige Luftspalte körperlich zu überbrücken. Die Wärmeleitmatte ist dabei gegenüber der Wärmeleitpaste bevorzugt, da Wärmeleitpaste durch schwer kontrollierbares Fließen in den Beatmungsgasstrom gelangen könnte. Die Wärmeleitmatte kann dagegen als Festkörper nicht fließen.

Da in der Regel der Antrieb des Gebläses während des Betriebs des Gebläses die leistungsstärkste Wärmequelle des Gebläses darstellt, ist bevorzugt ein den Antrieb des Gebläses umgebender Abschnitt des Gebläsegehäuses, etwa das oben genannte Antriebsgehäuseteil, der Gebläse-Verbindungsfläche gegenüberliegend, gegebenenfalls unter Zwischenanordnung von Wärmeleitpaste oder einer Wärmeleitmatte angeordnet. Zur Minimierung des Wärmeleitwiderstandes liegt eine Außenfläche des den Antrieb des Gebläses umgebenden Abschnitts des Gebläsegehäuses bevorzugt an der Gebläse-Verbindungsfläche an.

Ebenso kann der Wärmeleitkörper zur Sicherstellung eines möglichst stabilen Verbunds starr mit dem Gehäuse verbunden sein. Dies schafft nicht nur mechanische Stabilität und Robustheit, sondern stellt auch dauerhaft die Wärmeleitfähigkeit vom Gebläse bis zu einer Außenwand des Gehäuses des Notfall-Beatmungsgeräts sicher. Wiederum kann der Wärmeleitkörper einstückig mit einem Abschnitt des Gehäuses des Notfall-Beatmungsgeräts ausgebildet sein, was zwar für eine gegebene konstruktive Gestalt die bestmögliche, weil widerstandsärmste Wärmeleitung sorgt, jedoch die Gestaltungsfreiheit in der Gestaltung des Wärmeleitkörpers und gegebenenfalls auch des Gebläsegehäuses einschränkt. Bevorzugt ist daher der Wärmeleitkörper gesondert vom Gehäuse ausgebildet und mit diesem durch Verbindungsmittel verbunden, beispielsweise durch Schrauben, mit welchem eine Gehäuse-Verbindungsfläche, mit der der Wärmeleitkörper dem Gehäuse zugewandt ist, gegen eine Wandfläche des Gehäuses gespannt werden kann. Durch diese Spannmöglichkeit kann wiederum ein zwischen dem Wärmeleitkörper und dem Gehäuse etwaig vorhandener Spalt als Wärmeleitwiderstand minimiert werden.

Auch zwischen dem Wärmeleitkörper, insbesondere der Gehäuse-Verbindungsfläche, und dem Gehäuse kann Wärmeleitpaste oder eine Wärmeleitmatte zur Verringerung eines Wärmeleitwiderstandes angeordnet sein. Aus den oben genannten Gründen ist die Verwendung einer Wärmeleitmatte bevorzugt. Bevorzugt ist zur Erzielung möglichst geringer Wärmeleitwiderstände ein Spaltmaß zwischen Gebläse-Verbindungsfläche und Gebläsegehäuse sowie zwischen Gehäuse-Verbindungsfläche und Gehäuse jeweils geringer als 1 mm, stärker bevorzugt geringer als 0,3 mm.

Bei manchen Beatmungsgeräten wird von dem vom Gebläse geförderten Beatmungsgas ein Teilstrom als Kühlgasstrom abgezweigt und kühlbedürftigen Komponenten zugeführt. Dies ist an dem vorliegend diskutierten Notfall-Beatmungsgerät unnötig. Der vom Gebläse geförderte Beatmungsgasstrom kann ohne davon abgezweigtem Teilstrom vollständig dem Patienten zugeführt werden.

Allerdings kann auch an dem Notfall-Beatmungsgerät der vorliegenden Erfindung das vom Gebläse geförderte Beatmungsgas konvektiv Wärme vom Gebläse oder/und vom Wärmeleitkörper weg transportieren. Eine dadurch bewirkte Erwärmung des Beatmungsgases ist sogar erwünscht, da diese eine Kondensation von in Beatmungsgas gelöster Feuchtigkeit während eines Transports zum Patienten verhindert oder wenigstens verringert.

Zur Erzielung einer konvektiven Kühlung durch den Beatmungsgasstrom kann der Wärmeleitkörper wenigstens einen im Wärmeleitkörper geführten Kanal aufweisen, welcher wenigstens einen Teil eines Strömungswegs von der Umgebungsluft-Ansaugöffnung zur Beatmungsgas-Ausgabeöffnung derart bildet, dass der Kanal im Beatmungsbetrieb des Notfall-Beatmungsgeräts von von dem Gebläse zur Beatmungsgas-Ausgabeöffnung geförderten Beatmungsgas durchströmbar ist. Auch der den Kanal durchströmende Teil des Beatmungsgases, wobei vorzugsweise das gesamte Beatmungsgas den Kanal durchströmt, wird dem Patienten als Beatmungsgas zugeführt. Der Kanal kann mehrere Teilkanäle umfassen.

Dann, wenn der Wärmeleitkörper in besonders wirtschaftlicher Weise durch Strangpressen oder Strangextrudieren hergestellt ist, kann der wenigstens eine Kanal bereits beim Strangpressen oder beim Strangextrudieren geformt werden. Ein solcher Kanal muss jedoch nicht ausgeformt sein.

Zusätzlich oder alternativ kann der vom Gebläse geförderte Beatmungsgasstrom unmittelbar Wärme vom Gebläse abführen. Um dies in möglichst großem Umfang zu erreichen, ist erfindungsgemäß vorgesehen, dass eine Außenfläche des Gebläsegehäuses in einem Abschnitt eines Strömungswegs von der Umgebungsluft-Ansaugöffnung zur Beatmungsgas-Ausgabeöffnung derart freiliegt, dass sie im Beatmungsbetrieb des Notfall-Beatmungsgeräts von von dem Gebläse geförderten Beatmungsgas benetzbar ist. Der vom Beatmungsgas benetzbare Flächenanteil der Außenfläche des Gebläsegehäuses dient dann unmittelbar zur konvektiven Abgabe von Wärme an das den benetzten Flächenanteil anströmende Beatmungsgas.

Da das Beatmungsgas einen Abschnitt des Gebläsegehäuses, nämlich den eine Förderformation des Luftförderers aufnehmenden Abschnitt des Gebläsegehäuses, ohnehin durchströmt, weist ein den Luftförderer einhausender Abschnitt des Gebläsegehäuses eine im Verhältnis zum übrigen Gebläsegehäuse großen Flächenanteil auf, welcher effektiv Wärme an Gas übertragen kann, das an ihm vorbeiströmt und ihn dabei benetzt. Bevorzugt ist daher der vom Beatmungsgas benetzbare Teil des Gebläsegehäuses auf zwei entgegengesetzten Seiten, nämlich auf dessen Innenseite und auf dessen Außenseite von Beatmungsgas benetzbar. Daher ist, wie oben bereits dargelegt ist, bevorzugt ein den Antrieb umgebender Teil des Gebläsegehäuses, etwa das oben genannte Antriebsgehäuseteil, mit dem Wärmeleitkörper verbunden und optional in eine Ausnehmung des Wärmeleitkörpers eingesenkt. Weiter befindet sich ein den Luftförderer beweglicher Teil des Gebläsegehäuses in einem mit Beatmungsgas gefüllten Volumenbereich, etwa in einer Mischkammer oder/und in einem Strömungsraum zur Durchströmung mit Beatmungsgas, wenn dieses von der Umgebungsluft-Ansaugöffnung oder/und von dem Sondergas-Kopplungsabschnitt oder/und von dem Sondergas-Hilfseinlass zur Beatmungsgas-Ausgabeöffnung gefördert wird.

Dann, wenn der Luftförderer, wie es bevorzugt ist, ein relativ zum Gebläsegehäuse rotatorisch beweglicher Luftförderer ist, ist zumindest ein von Beatmungsgas benetzbarer Flächenbereich der Außenseite des Gebläsegehäuses ein eine Drehachse des Luftförderers umgebender, bevorzugt vollständig längs einer geschlossenen Bahn umgebender, Flächenbereich. So können in der Außenumgebung des Gebläsegehäuses durch die Bewegung des Luftförderers entstehende Wirbel für eine Erhöhung der konvektiv abgeführten Wärmemenge genutzt werden.

Wenigstens ein Abschnitt des Gebläsegehäuses bildet somit eine Wandung eines ein Beatmungsgas umschließenden Bereichs, also etwa eine Wandung der Mischkammer oder/und des Strömungsraums des Beatmungsgases. Es sei an dieser Stelle angemerkt, dass auch die Mischkammer Teil eines Strömungsraums des Beatmungsgases ist, da auch das in der Mischkammer befindliche Beatmungsgas zur Beatmungsgas-Ausgabeöffnung gefördert wird.

Ein Abschnitt der Oberfläche des Wärmeleitkörpers bildet erfindungsgemäß eine Wandung eines ein Beatmungsgas umschließenden Bereichs, also etwa eine Wandung der Mischkammer oder/und des Strömungsraums des Beatmungsgases. So kann der Wärmeleitkörper, in welchen vom Gebläse, insbesondere vom Gebläseantrieb, Wärme eingeleitet wird, nicht nur durch das Gehäuse zur Umgebung hin gekühlt werden, sondern auch durch erzwungene Konvektion mittels des geförderten Beatmungsgases.

Der Wärmeleitkörper kann nicht nur für eine Wärmeabfuhr aus dem Gehäuse sorgen, sondern kann als Strukturbauteil zusätzlich der Notfall-Beatmungskammer Stabilität verleihen. Hierzu umgibt der Wärmeleitkörper erfindungsgemäß einen Abschnitt des Strömungswegs des Beatmungsgases, etwa die oben genannte Mischkammer, von wenigstens drei Seiten, vorzugsweise von vier Seiten, unter Bildung einer Ausnehmung. In dieser Ausnehmung ist dann erfindungsgemäß der von Beatmungsgas benetzbare Teil der Außenfläche des Gebläsegehäuses angeordnet, um sicherzustellen, dass der benetzbare Teil der Außenfläche während eines möglichst großen Zeitanteils des Betriebs des Notfall-Beatmungsgeräts von Beatmungsgas auch tatsächlich benetzt ist.

Um gewährleisten zu können, dass auch das Gebläsegehäuse ausreichend gute Wärmeleiteigenschaften hat, um Wärme aus dem Inneren des Gebläsegehäuses nach außen oder/und von einem den Gebläseantrieb einfassenden Bereich in einen den Luftförderer einfassenden Bereich zu leiten, ist es bevorzugt, dass das Gebläsegehäuse ein Material umfasst oder aus einem Material gebildet ist, welches ausgewählt ist aus Leichtmetall, Buntmetall oder/und einem mit wärmeleitfähigem Füllmaterial gefüllten Kunststoff.

Bevorzugt kann das Gebläsegehäuse wenigstens abschnittsweise, bevorzugt vollständig, aus dem gleichen Material gebildet sein wie der Wärmeleitkörper.

Ebenso ist es für eine ausreichende Wärmeleitung des Gehäuses des Notfall-Beatmungsgeräts vorteilhaft, wenn das Gehäuse ein Material umfasst oder aus einem Material gebildet ist, welches ausgewählt ist aus Leichtmetall, Buntmetall oder/und einem mit wärmeleitfähigem Füllmaterial gefüllten Kunststoff. Das Gehäuse kann zu Erleichterung von Fertigung und Montage aus dem gleichen Material gebildet sein wie der Wärmeleitkörper.

Das Leichtmetall kann Aluminium, Magnesium, eine Aluminiumlegierung oder/und eine Magnesiumlegierung umfassen. Diese Leichtmetalle weisen eine gute Wärmeleitfähigkeit bei verhältnismäßig geringer Dichte und ausreichend hoher Festigkeit sowie geringer Korrosionsneigung auf.

Das Buntmetall kann Kupfer oder/und eine Kupferlegierung umfassen. Bekannte und taugliche Kupferlegierungen sind beispielsweise Bronze und Messing, wobei deren genaue Zusammensetzung jeweils über einen Breitenbereich variieren kann. Kupfer und Kupferlegierungen weisen eine außergewöhnlich hohe Wärmeleitfähigkeit auf, haben jedoch auch eine höhere Dichte und eine höhere Korrosionsneigung als Leichtmetalle.

Das wärmeleitfähige Füllmaterial kann ein Metall, beispielsweise ein Leichtmetall im obigen Sinne, oder/und eine Keramik oder/und Graphit oder/und Kohlenstoff, insbesondere Kohlenstoff-Nanoröhrchen, umfassen. Eine bekannte Keramik, welche bei verhältnismäßig geringer Dichte die Wärmeleitfähigkeit einer Kunststoffmatrix, in der die Keramik eingebettet ist, erhöht, ist Bornitrid. Grundsätzlich kann jedes Material als geeignetes wärmeleitfähiges Füllmaterial dienen, welches eine Wärmeleitfähigkeit vom mindestens 14 W/(mK) aufweist.

Das Füllmaterial kann beispielsweise in Partikel- oder Faserform in einer Kunststoffmatrix aufgenommen sein. Der Kunststoff kann ein Duroplast oder Thermoplast sein. Zur erleichterten Formgebung ist der Kunststoff bevorzugt ein Thermoplast.

Oben wurde bereits die Gebläse-Verbindungsfläche des Wärmeleitkörpers erwähnt, welche einem Abschnitt des Gebläsegehäuses gegenüberliegt, und ebenso die Gehäuse-Verbindungsfläche des Wärmeleitkörpers, welche zu einer Innenfläche des Gehäuses hinweist. Für eine effektive Wärmeausleitung aus dem Notfall-Beatmungsgerät ist es vorteilhaft, wenn vom Wärmeleitkörper transportierte Wärme über eine möglichst große Fläche an das Gehäuse des Notfall-Beatmungsgeräts übertragen werden kann. Daher ist die Gehäuse-Verbindungsfläche wenigstens doppelt so groß, vorzugsweise wenigstens viermal so groß, besonders bevorzugt wenigstens sechsmal so groß ist wie die Gebläse-Verbindungsfläche.

Bevorzugt hat das Gehäuse des vorliegenden Notfall-Beatmungsgeräts eine einfache Gehäusegestalt, vorzugsweise mit einer prismatischen oder/und zylindrischen Grundform. Somit hat das Gehäuse bevorzugt zwei zueinander im Wesentlichen parallele Stirnseiten und eine die beiden Stirnseiten miteinander verbindende Mantelfläche. Die Mantelfläche läuft um eine virtuelle, die Stirnseiten miteinander verbindende Prismenachse um. Die Mantelfläche kann dabei polyedrisch mit in Umlaufrichtung um die Prismenachse aufeinander folgenden ebenen Flächen ausgestaltet sein. Zur Vermeidung von Verletzungen sind die Verbindungsbereiche zwischen zwei in Umlaufrichtung unmittelbar benachbarten ebenen Mantelflächenabschnitten abgerundet. Bevorzugt weist der Krümmungsradius eines solchen Verbindungsabschnitts wenigstens 0,5 cm auf. Die Krümmungsachse ist vorzugsweise parallel zur Prismenachse. Die Mantelfläche kann ebenso zylindrisch ausgebildet sein, wobei die Querschnittsfläche der zylindrischen Grundform kreisförmig oder elliptisch sein kann.

Zur Erzielung einer möglichst großen Gehäuse-Verbindungsfläche liegt diese bevorzugt an einem die Mantelfläche ausbildenden Wandabschnitt des Gehäuses an, gegebenenfalls unter Zwischenanordnung der oben erwähnten Wärmeleitpaste oder Wärmeleitmatte.

Gemäß einer weiteren vorteilhaften Ausführungsform der vorliegenden Erfindung kann der Wärmeleitkörper mehrteilig ausgebildet sein. Beispielsweise kann der Wärmeleitkörper ein gebläsegehäuseseitiges Körperteil und ein vom gebläsegehäuseseitigen Körperteil gesondert ausgebildetes gehäuseseitiges Körperteil aufweisen. Das gebläsegehäuseseitige Körperteil kann die oben genannte Gebläse-Verbindungsfläche aufweisen und kann in einer der oben beschriebenen weisen Wärme übertragend mit dem Gebläsegehäuse verbunden sein. Das gehäuseseitige Körperteil kann die oben genannte Gehäuse-Verbindungsfläche aufweisen und in einer der oben beschriebenen weisen Wärme übertragend mit dem Gehäuse des Beatmungsgeräts verbunden sein.

Bei einer einstückigen Ausbildung des Wärmeleitkörpers sind die Gebläse-Verbindungsfläche und die Gehäuse-Verbindungsfläche in unterschiedlichen Bereichen ein und desselben einstückigen Wärmeleitkörpers ausgebildet und Wärme zwischen diesen Bereichen wird überwiegend konduktiv durch das Material des Wärmeleitkörpers übertragen.

Bei der mehrteiligen Ausbildung können die genannten Körperteile durch wenigstens eine Heatpipe, vorzugsweise durch mehrere Heatpipes, Wärme übertragend miteinander in Verbindung stehen. Zur Klarstellung weisen wir darauf hin, dass anstelle des gängigen Begriffs "Heatpipe" bisweilen in der Literatur auch der Begriff "Wärmerohr" verwendet wird. Bei einer Heatpipe wird im allgemeinen in einem in der Regel länglichen abgeschlossenen Gefäß, dem Rohr, ein Fluid in einer Wärmeaufnahmezone verdampft und in einer Wärmeabgabezone kondensiert. Eine interne Struktur der Heatpipe, häufig unter Ausnutzung von Kapillarwirkung, sorgt für einen Transport des Fluids in unterschiedlichen Aggregatszuständen zwischen der Wärmeabgabezone und der Wärmeaufnahmezone.

Vorliegend ist bevorzugt die Wärmeaufnahmezone der wenigstens einen Heatpipe im gebläsegehäuseseitigen Körperteil angeordnet und ihre Wärmeabgabezone im gehäuseseitigen Körperteil. In einem Bereich zwischen den beiden genannten Körperteilen kann die wenigstens eine Heatpipe freiliegen oder einen anderen Körper ganz oder teilweise durchsetzen.

Die wenigstens eine Heatpipe ist bevorzugt wenigstens bereichsweise gekrümmt, um den Bauraum im Gehäuse, also im Gehäuse des Beatmungsgeräts, möglichst effektiv zur Aufnahme der Heatpipe nutzen zu können. Für eine besonders einfache Montage weist die wenigstens eine Heatpipe eine im Wesentlichen geradlinige Wärmeaufnahmezone und ebenso eine im Wesentlichen geradlinige Wärmeabgabezone auf und weist eine zwischen den genannten Zonen gelegene gekrümmte Zone auf. Für das vorliegend beschriebene Beatmungsgerät hatte sich als vorteilhaft herausgestellt, wenn die Längsachsen der Wärmeaufnahmezone und der Wärmeabgabezone der wenigstens einen Heatpipe einen Winkel von zwischen 70 und 110°, bevorzugt zwischen 80 und 100°, besonders bevorzugt von 90°, aufweisen. Für den bevorzugten Fall, dass mehrere Heatpipes zur wärmeübertragenden Verbindung der beiden Körperteile des Wärmeleitkörpers angeordnet sind, sind vorzugsweise gleichartige Heatpipes parallel nebeneinander angeordnet.

Zur möglichst effektiven Wärmeübertragung vom gebläsegehäuseseitigen Körperteil zur Wärmeaufnahmezone der Heatpipe und von der Wärmeabgabezone der Heatpipe zum gehäuseseitigen Körperteil sind die jeweiligen Verbindungen von Körperteil und Zone der Heatpipe bevorzugt durch Lötverbindungen gebildet. So können Luftspalte als Wärmeübergangswiderstände vermieden werden.

Die Mantelfläche kann auch sowohl prismatisch als auch zylindrisch sein, wenn sie etwa längs eines ersten Umfangsabschnitts polyedrisch und längs eines daran anschließenden zweiten Umfangsabschnitts zylindrisch bzw. teilzylindrisch ausgebildet ist. Zu Erleichterung der Montage des Wärmeleitkörpers ist die Gehäuse-Verbindungsfläche bevorzugt eben.

Bevorzugt ist das Gebläse zur Förderung von Beatmungsgas das einzige Gebläse bzw. der einzige Gasförderer im Notfall-Beatmungsgerät, sodass gesonderte Kühlerlüfter entfallen können.

Die vorliegende Erfindung wird nachfolgend anhand der beiliegenden Zeichnungen beschrieben werden. Es stellt dar:
- Figur 1: eine perspektivische Explosionsansicht einer ersten Ausführungsform eines erfindungsgemäßen Notfall-Beatmungsgeräts,
- Figur 2: eine Längsschnittansicht durch die erste Ausführungsform des erfindungsgemäßen Notfall-Beatmungsgeräts von Figur 1, mit einer Schnittebene parallel zu den Flächen 14b und 14d in Fig. 1,
- Figur 3: eine Draufsicht auf die von einem abnehmbaren Gehäusedeckel gebildete eine Stirnseite des Notfall-Beatmungsgeräts der Figuren 1 und 2,
- Figur 4: eine Draufsicht auf die andere, entgegengesetzte Stirnseite des NotfallBeatmungsgeräts der Figuren 1 und 2,

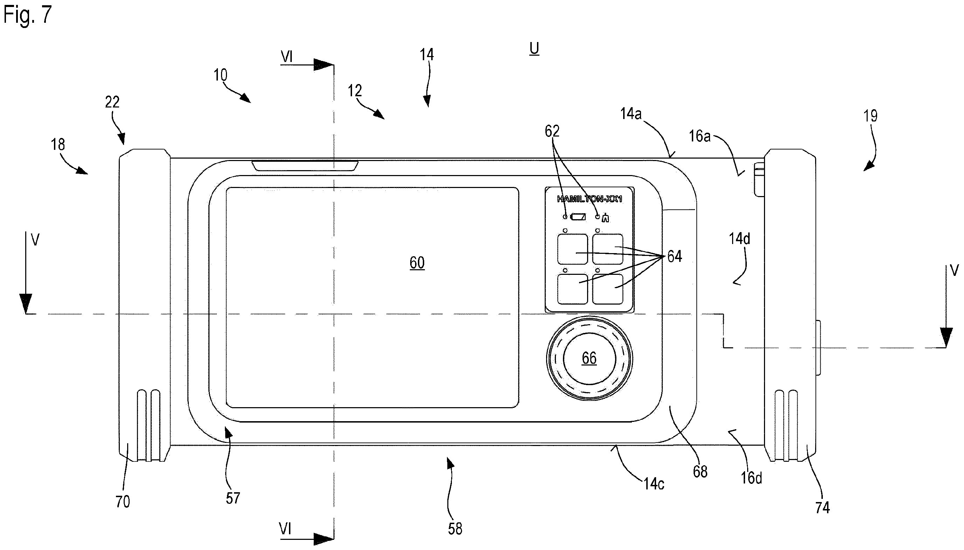

- Figur 5: eine Längsschnittansicht entlang der Schnittfläche V-V von Figur 7,
- Figur 6: eine Querschnittansicht entlang der zur Prismenachse P orthogonalen Schnittebene VI-VI von Figur 7,
- Figur 7: eine Draufsicht auf die ebene Vorderfläche 14d mit der Eingabe-/AusgabeVorrichtung 58 des Notfall-Beatmungsgeräts von Figur 1, und
- Figur 8: eine der Perspektive von Figur 6 entsprechende Querschnittsansicht einer zweiten Ausführungsform eines erfindungsgemäßen Notfall-Beatmungsgeräts, bei Betrachtung in einer zur Prismenachse P orthogonalen Schnittebene.

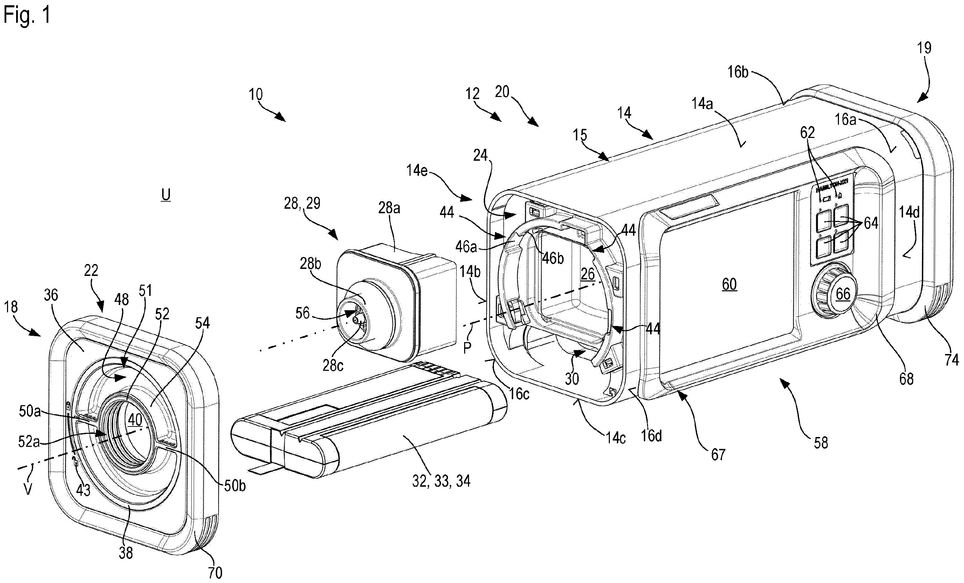

In Figur 1 ist eine erste erfindungsgemäße Ausführungsform eines Notfall-Beatmungsgeräts allgemein mit 10 bezeichnet. Das Notfall-Beatmungsgerät 10 umfasst ein Gehäuse 12 mit prismatischer Grundform, vorliegend mit quaderartiger Grundform.

Die Mantelfläche 14 des Gehäuses 12 umfasst vier ebene Flächenanteile 14a, 14b, 14c und 14d, von welchen jeweils in Umlaufrichtung um die Prismenachse P aufeinanderfolgende ebene Flächenanteile 14a, 14b, 14c und 14d zueinander orthogonal orientiert sind. Alle ebenen Flächenanteile 14a, 14b, 14c und 14d sind zur Prismenachse P parallel. Die ebenen Flächenanteile 14a, 14b, 14c und 14d sind durch viertelzylindrische Flächenanteile 16a, 16b, 16c und 16d miteinander bevorzugt fügestellenfrei verbunden. Die einzelnen Zylinderachsen der viertelzylindrischen und damit gekrümmten Flächenanteile 16a, 16b, 16c und 16d sind parallel zur Prismenachse P. Bevorzugt ist das die Mantelfläche 14 aufweisende Gehäusebauteil 15 ein extrudiertes Aluminiumrohr.

An der dem Betrachter von Figur 1 zugewandten Stirnseite 18 des Gehäuses 12 umfasst das Gehäuse 12 einen vom übrigen Gehäuse 20 längs der Prismenachse P abnehmbaren und am übrigen Gehäuse 20 anordenbaren Gehäusedeckel 22. Der Gehäusedeckel 22 dient somit zum Verschließen einer an dem dem Betrachter von Figur 1 näher gelegenen Längsende 14e der Mantelfläche 14 gebildeten Gehäuseöffnung 24. Die Gehäuseöffnung 24 ist durch die Mantelfläche 14 des Restgehäuses 20 begrenzt. Durch die Gehäuseöffnung 24 ist ein Filter-Aufnahmefach 26 für eine Luftfilter-Kartusche 28 mit einem Luftfilter 29 und ist ein Akku-Aufnahmefach 30 für einen wiederaufladbaren elektrischen Akkumulator 32 als einen netzunabhängigen Energiespeicher 34 zugänglich.

Der Gehäusedeckel 22 weist ein Deckelbauteil 36 und ein Verriegelungsbauteil 38 auf. Das Verriegelungsbauteil 38 ist am Deckelbauteil 36 um die Verriegelungsachse V drehbar gelagert. Die Verriegelungsachse V verläuft im Verschlusszustand, also dann, wenn der Gehäusedeckel 22 am Restgehäuse 20 angeordnet ist und die Gehäuseöffnung 24 verschließt, koaxial mit der Prismenachse P.

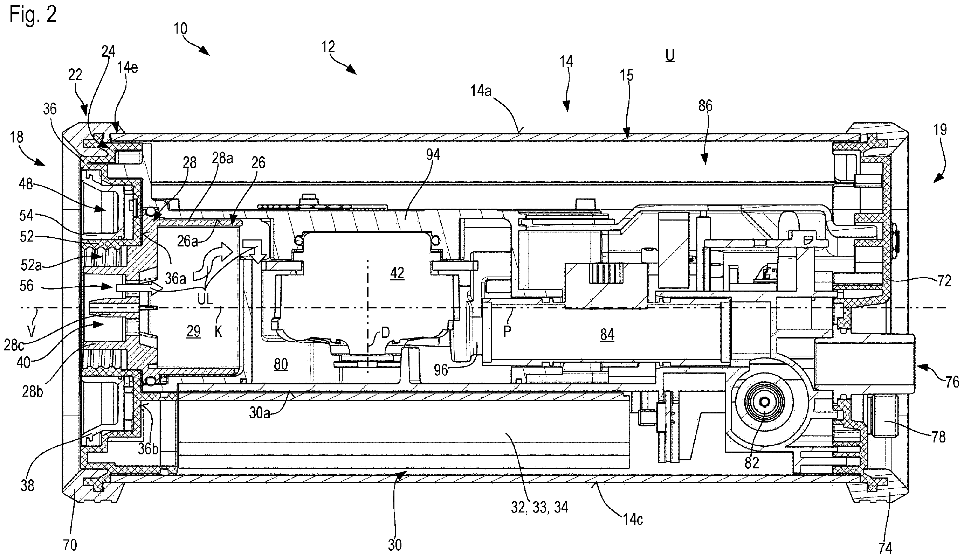

Der Gehäusedeckel 22 weist außerdem eine Umgebungsluft-Ansaugöffnung 40 auf, welche sowohl das Deckelbauteil 36 als auch das Verriegelungsbauteil 38 durchsetzt. Durch die Umgebungsluft-Ansaugöffnung 40 hindurch kann von einem Gebläse 42 (siehe Figur 2) Umgebungsluft aus der Umgebung U durch den Luftfilter 29 hindurch in das Gehäuse 12 angesaugt werden.

Das Verriegelungsbauteil 38 ist in Figur 1 in seiner Verriegelungsstellung gezeigt, von welcher es entgegen dem Uhrzeigersinn etwa um eine Zwölftel-Umdrehung um die Verriegelungsachse V in eine durch ein Symbol 43 in Gestalt eines offenen Vorhängeschlosses gekennzeichnete Freigabestellung drehbar ist. Das Verriegelungsbauteil 38 weist radial in Richtung von der Verriegelungsachse V weg vorstehende Vorsprünge auf, welche in Figur 1 durch das Deckelbauteil 36 verdeckt sind. Diese Vorsprünge sind Teil einer Bajonettverriegelung, durch welche der die Gehäuseöffnung 24 verschließende Gehäusedeckel 22 an einer relativ zum Restgehäuse 20 unbeweglichen Verriegelungsgegenformation 44 formschlüssig verriegelbar ist. Die Verriegelungsgegenformation 44 weist hierzu mehrere Ausnehmungen 46 auf, jeweils mit einem axialen Ausnehmungsabschnitt 46a und mit einem Ausnehmungsabschnitt 46b in Umfangsrichtung um die Verriegelungsachse V. Die Vorsprünge des Verriegelungsbauteils 38 können dann, wenn es sich in der Freigabestellung befindet, längs des axialen Ausnehmungsabschnitts 46a parallel zur Verriegelungsachse V und damit parallel zur Prismenachse P zum Ausnehmungsabschnitt 46b hingeführt werden und nach Erreichen des Ausnehmungsabschnitts 46b in Umfangsrichtung entlang des Ausnehmungsabschnitts 46b bewegt werden.

Das Verriegelungsbauteil 38 weist eine in Umlaufrichtung verlaufende Griffmulde 48 auf, welche durch zwei Griffstege 50a und 50b unterbrochen ist, die einander diametral bezüglich der zwischen ihnen gelegenen Umgebungsluft-Ansaugöffnung 40 gegenüberliegen. Durch Handangriff an die Griffstege 50a und 50b kann sowohl das Verriegelungsbauteil 38 zwischen der Freigabestellung und der Verriegelungsstellung verdreht als auch der freigegebene Gehäusedeckel 22 längs der Prismenachse P vom Restgehäuse 20 abgehoben oder auf dieses aufgesetzt werden. Die Griffstege 50a und 50b sowie die Griffmulde 48 bilden gemeinsam eine Betätigungsformation 51 zur Betätigung des Verriegelungsbauteils 38.

Der Gehäusedeckel 22 ist daher durch einhändige Bedienung sowohl vom Restgehäuse 20 abnehmbar als auch auf dieses aufsetzbar als auch in der Verschlussstellung verriegelbar und freigebbar.

Die Umgebungsluft-Ansaugöffnung 40 ist nach radial außen - bezogen auf die Verriegelungsachse V - unmittelbar durch einen Lagerabschnitt 52 des Deckelbauteils 36 begrenzt. Der Lagerabschnitt 52 weist eine Befestigungsformation 52a in Form eines Innengewindes auf. An dieser Befestigungsformation 52a kann beispielsweise ein zusätzlicher Luftfilter angeordnet werden, welcher Filterfunktionen erfüllt, die der Luftfilter 29 der Luftfilter-Kartusche 28 nicht leistet. Alternativ oder zusätzlich kann an der Befestigungsformation 52a eine Messvorrichtung angeordnet werden, welche die Umgebungsluft-Ansaugöffnung 40 durchströmende angesaugte Umgebungsluft messtechnisch erfasst, etwa deren chemische Zusammensetzung bestimmt oder bestimmt, ob und gegebenenfalls in welchem Ausmaß die angesaugte Umgebungsluft einen vorbestimmten Bestandteil enthält oder nicht.

Der Lagerabschnitt 52 ist radial außen von einem Lagergegenabschnitt 54 des Verriegelungsbauteils 38 umgeben. Der Lagerabschnitt 52 wirkt gleichsam als Achsbauteil, welches das Verriegelungsbauteil 38 mittels dessen Lagergegenabschnitts 54 um die Verriegelungsachse V drehbar lagert. Der Lagergegenabschnitt 54 bildet eine radial innere Grenze der Griffmulde 48.

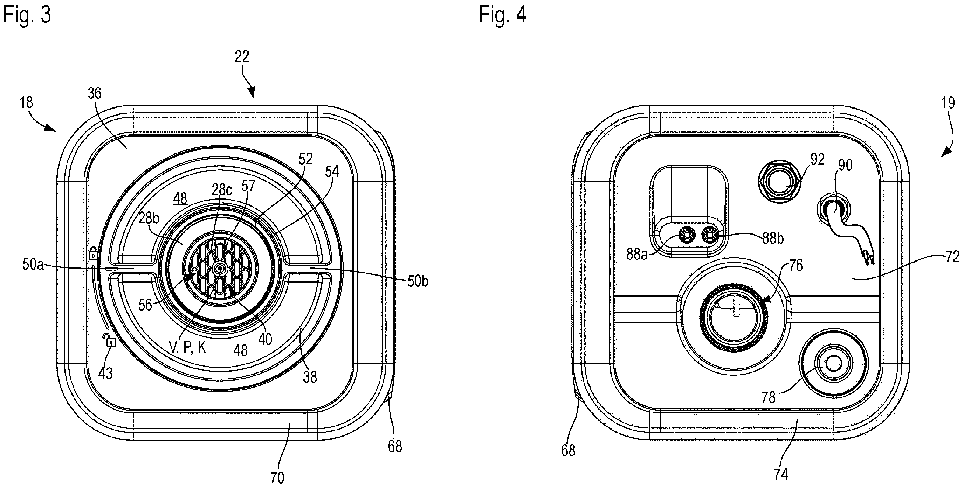

Die Luftfilter-Kartusche 28 weist auf ihrer im Betrieb dem Gehäusedeckel 22 zugewandten Seite eine Umgebungsluft-Einlassöffnung 56 auf, welche von einem vom Kartuschenhauptkörper 28a auskragend abstehenden Kragen 28b eingefasst ist. Eine den Kragen 28b zentral durchsetzend gedachte Kartuschen-Einlassachse K ist im betriebsbereiten Zustand des Notfall-Beatmungsgeräts 10 koaxial zur Verriegelungsachse V und zur zentral die Mantelfläche 14 durchsetzend gedachten virtuellen Prismenachse P. Die Umgebungsluft-Einlassöffnung 56 ist durch ein Schutzgitter 57 (siehe Figur 3) vor einem Eintritt größerer Schmutzpartikel, wie Steine, Wollmäuse und dgl., geschützt. Das Schutzgitter 57 kann spritzgusstechnisch einstückig mit einem die Umgebungsluft-Einlassöffnung aufweisenden Gehäuseteil der Luftfilter-Kartusche 28 ausgebildet sein.

Konzentrisch zu dem Kragen 28b ragt längs der Kartuschen-Einlassachse K ein Sondergas-Hilfseinlass 28c in Gestalt eines sich vom Kartuschenhauptkörper 28a weg verjüngenden auskragenden Anschlussstutzens ab. Eine Sondergasversorgung, beispielsweise eine Sauerstoff-Hilfsversorgung kann an den Sondergas-Hilfseinlass 28c schnell und unkompliziert angeschlossen werden, beispielsweise indem ein in seinem Durchmesser ausreichend klein bzw. groß bemessener elastischer Schlauch auf den Sondergas-Hilfseinlass 28c aufgeschoben und reibschlüssig dort gehalten wird. Durch die sich vom Kartuschenhauptkörper 28a weg verjüngende Gestalt des Sondergas-Hilfseinlasses 28c können Schläuche in einem vorbestimmten Durchmesserbereich ausreichend sicher kurzfristig mit dem Sondergas-Hilfseinlass 28c verbunden werden.

Der Energiespeicher 34 weist in dem dargestellten bevorzugten Ausführungsbeispiel einen einzigen Energiespeicherkörper 33 auf.

Auf dem ebenen Flächenanteil 14d und ausgehend davon sich in die Teil zylindrischen benachbarten Flächenanteile 16d und 16a erstreckend weist das Notfall-Beatmungsgerät 10 eine Eingabe-/Ausgabe-Vorrichtung 58 auf, welche dem Informationsaustausch zwischen Bedienperson und Notfall-Beatmungsgerät 10 dient und welche der Steuerung des Notfall-Beatmungsgeräts 10 durch die Bedienperson dient. Die Eingabe-/Ausgabe-Vorrichtung 58 weist einen Bildschirm 60 als eine Ausgabevorrichtung auf, welcher vorzugsweise ein Touchscreen ist, der berührungssensitiv die Eingabe von Information gestattet. Die Eingabe-/Ausgabe-Vorrichtung 58 weist darüber hinaus Anzeige-LEDs 62 als weitere Ausgabevorrichtung auf und weist beispielhaft Tastschalter 64 und einen Drehschalter 66 als Eingabemittel auf.

Zum Schutz vor stoßartigen Belastungen kann die Eingabe-/Ausgabe-Vorrichtung 58 durch ein Fassungsbauteil 67, in beispielhafter Ausgestaltung als stoßdämpfender Elastomerring 68, etwa aus Gummi, Kautschuk und dergleichen, umgeben sein. Das die Eingabe-/Ausgabe-Vorrichtung 58 umgebende Fassungsbauteil 67 kann jedoch auch als Kunststoffspritzgussbauteil aus einem thermoplastischen Kunststoff gebildet sein.

Auch der Gerätedeckel 22 ist durch einen in Umlaufrichtung vollständig um die Prismenachse P umlaufenden stoßdämpfenden Elastomerring 70 umgeben. Im Verschlusszustand bedeckt der Elastomerring 70 ebenso einen Teil der Mantelfläche 14 wie der Stirnseite 18, sodass der Elastomerring 70 im Bereich des Gerätedeckels 22 das Notfall-Beatmungsgerät 10 sowohl gegen axiale als auch gegen radiale Stoßbelastungen schützt.

An dem dem Gerätedeckel 22 entgegengesetzten Längsende 14f der Mantelfläche 14 ist ebenfalls ein Gerätedeckel 72 (siehe Figur 2) angeordnet. Im Gegensatz zum Gerätedeckel 22 ist der Gerätedeckel 72 jedoch bevorzugt nicht von der Mantelfläche 14 des Gehäuses 12 abnehmbar. Um auch das Längsende des Gerätedeckels 72 vor axialen und radialen Stoßbelastungen zu schützen ist auch an diesem Längsende ein vollständig geschlossen in Umlaufrichtung um die Prismenachse P umlaufender Elastomerring 74 vorgesehen, welcher sowohl einen Teil der Mantelfläche 14 als auch einen Teil der Stirnseite 19 bedeckt. Die Stirnseite 19 ist der Stirnseite 18 entgegengesetzt.

Die Elastomerringe 68, 70 und 74 sind zur fertigungstechnischen Vereinfachung bevorzugt aus demselben weichelastischen Material gebildet.

Figur 2 zeigt einen Längsschnitt durch das Notfall-Beatmungsgerät 10 längs einer Schnittebene, welche die Prismenachse P enthält und parallel zu den ebenen Flächenanteilen 14d und 14b verläuft.

Wie in dem in Figur 2 gezeigten betriebsbereiten Verschlusszustand des Notfall-Beatmungsgeräts 10 erkennbar ist, weist das Deckelbauteil 36 einen Deckel-Filter-Positionierabschnitt 36a auf, welcher im Verschlusszustand in Anlageeingriff ist mit einem Abschnitt der Luftfilter-Kartusche 28, insbesondere mit dem Kartuschenhauptkörper 28a und so zur definierten Lage der Luftfilter-Kartusche 28 und des Luftfilters 29 im Gehäuse 12 beiträgt. Weiter weist das Notfall-Beatmungsgerät 10 einen Gehäuse-Filter-Positionierabschnitt 26a auf, beispielsweise in Gestalt einer Innenwand des Filter-Aufnahmefachs 26. Im Zusammenwirken definieren der Deckel-Filter-Positionierabschnitt 36a und der Gehäuse-Filter-Positionierabschnitt 26a die Betriebsposition der Luftfilter-Kartusche 28 ausreichend genau.

Ebenso weist das Deckelbauteil 36 einen Deckel-Speicher-Positionierabschnitt 36b auf, welche im dargestellten Verschlusszustand in Anlageeingriff ist mit dem Energiespeicherkörper 33 und den Energiespeicherkörper 33 im Zusammenwirken mit einem Gehäuse-Speicher-Positionierabschnitt 30a, etwa einer Innenwand des Akku-Aufnahmefachs 30, ausreichend genau in seiner Betriebsposition fixiert.

Auf der Stirnseite 19 im gehäusefesten Gehäusedeckel 72 liegt die Beatmungsgas-Ausgabeöffnung 76 (siehe auch Figur 4), durch welche hindurch vom Gebläse 42 gefördertes inspiratorisches Beatmungsgas aus dem Gehäuse 12 zu einem an das Notfall-Beatmungsgerät 10 angeschlossenen Patienten hin austritt.

Hinter der Schnittebene von Figur 2, unterhalb der Beatmungsgas-Ausgabeöffnung 76 ist, wiederum im gehäusefesten Gehäusedeckel 72, ein Sondergas-Kopplungsabschnitt 78, etwa ein Sondergas-Anschlussstutzen, vorgesehen, durch welchen ebenfalls ein von Umgebungsluft verschiedenes Sondergas in das Notfall-Beatmungsgerät 10 eingeleitet werden kann. Auch dieses Sondergas kann beispielsweise Sauerstoff sein.

Somit gestattet das Notfall-Beatmungsgerät 10 die Mischung eines Beatmungsgases aus drei verschiedenen Gasen, nämlich aus Umgebungsluft, aus einem durch den Sondergas-Kopplungsabschnitt 78 eingeleiteten ersten Sondergas und aus einem durch den Sondergas-Hilfseinlass 28c eingeleiteten zweiten Sondergas. Sofern nur ein weiteres von Umgebungsluft verschiedenes Sondergas zur Mischung des Beatmungsgases benötigt wird, wird dieses bevorzugt über den Sondergas-Kopplungsabschnitt 78 eingeleitet.

Durch die Umgebungsluft-Ansaugöffnung 40 angesaugte Umgebungsluft UL tritt, wie durch die weiß gefüllten Pfeile in Figur 2 dargestellt, durch die Umgebungsluft-Einlassöffnung 56 in den Kartuschenhauptkörper 28a ein, tritt durch den Luftfilter 29 hindurch und erreicht eine Mischkammer 80, in welcher das Gebläse 42 mit seiner Ansaugöffnung angeordnet ist. Das in der Mischkammer 80 vorhandene Gas benetzt einen Großteil der Außenfläche des Gebläses 42 und trägt so zu dessen konvektiver Kühlung bei.

Ein durch den Sondergas-Kopplungsabschnitt 78 eingeleitetes Sondergas, beispielsweise Sauerstoff, kann in seinem Mengenstrom durch ein verstellbares Proportionalventil 82 über die Eingabe-/Ausgabe-Vorrichtung 58 passend eingestellt werden und erreicht über eine Sondergas-Zufuhrleitung 84 ebenfalls die Mischkammer 80, wo sich die Umgebungsluft UL und dass Sondergas bereits vor Eintritt in das Gebläse 42 vermischen können. Das Gebläse 42 dient vorliegend also nicht nur zur Förderung des Beatmungsgases sondern auch zu dessen möglichst homogener Durchmischung, sodass ein möglichst homogenes Beatmungsgas aus der Beatmungsgas-Ausgabeöffnung 76 austritt. Die Förderleitung, welche druckseitig das Beatmungsgas vom Gebläse 42 zur Beatmungsgas-Ausgabeöffnung 76 leitet, liegt in Figur 2 hinter der Schnittebene der Figur 2 und liegt hinter einem Elektronik-Kompartiment 86, welches gegenüber der Sondergas-Zufuhrleitung 84 körperlich vollständig abgeschirmt ist, um jegliche Zündgefahr auszuschließen, die ein Funke, welcher in der im Elektronik-Kompartiment 86 aufgenommenen Elektronik entstehen könnte, oder auch nur ausreichende Hitze in einer Umgebung reinen Sauerstoffs oder stark erhöhten Sauerstoffgehalts haben könnte. Im Elektronik-Kompartiment 86 ist eine Steuervorrichtung zur Steuerung des Betriebs des Notfall-Beatmungsgeräts 10 aufgenommen.

In Figur 3 ist eine Draufsicht auf die Stirnseite 18 mit dem abnehmbaren Gehäusedeckel 22 dargestellt, also mit Blickrichtung längs der koaxialen Achsen Verriegelungsachse V, Prismenachse P und Kartuschen-Einlassachse K.

Figur 4 zeigt eine Draufsicht auf die Stirnseite 19 mit gehäusefesten Gehäusedeckel 72. Die Blickrichtung von Figur 4 ist jener von Figur 3 entgegengesetzt.

Über die bereits im Zusammenhang mit den Figuren 1 und 2 erläuterten Merkmale hinaus zeigt Figur 4 Anschlussstutzen 88a und 88b, an welche Druckerfassungsschläuche anschließbar sind, welche an ihrem von den Anschlussstutzen 88a bzw. 88b fernliegenden anderen Ende jeweils mit einem Innenbereich eines Differenzdruck-Flusssensors zur Messung eines proximalen inspiratorischen und bevorzugt auch exspiratorischen Beatmungsgasstroms verbunden sind. Die beiden Innenbereiche sind in an sich bekannter Weise durch einen Strömungswiderstand voneinander getrennt, wobei der Strömungswiderstand durch den Beatmungsgasstrom veränderlich ist.

Über einen Netzeingang 90 ist das Notfall-Beatmungsgerät 10 bei räumlicher Verfügbarkeit eines Netzanschlusses mit Energie aus einem öffentlichen Stromversorgungsnetz betreibbar. Alle elektrischen Funktionseinheiten des Notfall-Beatmungsgeräts 10 können dann mit Netzspannung versorgt werden, in der Regel unter Zwischenschaltung eines auf Niedervoltspannung transformierenden Netzteils im Gehäuse 12. Ebenso kann der Akkumulator 32 aufgeladen werden. Eine Buchse 92 im Gehäuse 12 ist zum Anschluss eines externen Sensors, insbesondere CO₂-Sensors, angeordnet. Ein solcher CO₂-Sensor kann beispielsweise an einem mit der Notfall-Beatmungsvorrichtung 10 gekoppelten Durchflusssensor vorgesehen und zu einer Sensoranordnung gekoppelt sein.

In den Schnittansichten der Figuren 2, 5 und 6 ist in Schnittansicht ein Wärmeleitkörper 94 dargestellt, an welchem das Gebläse 42 gehaltert ist.

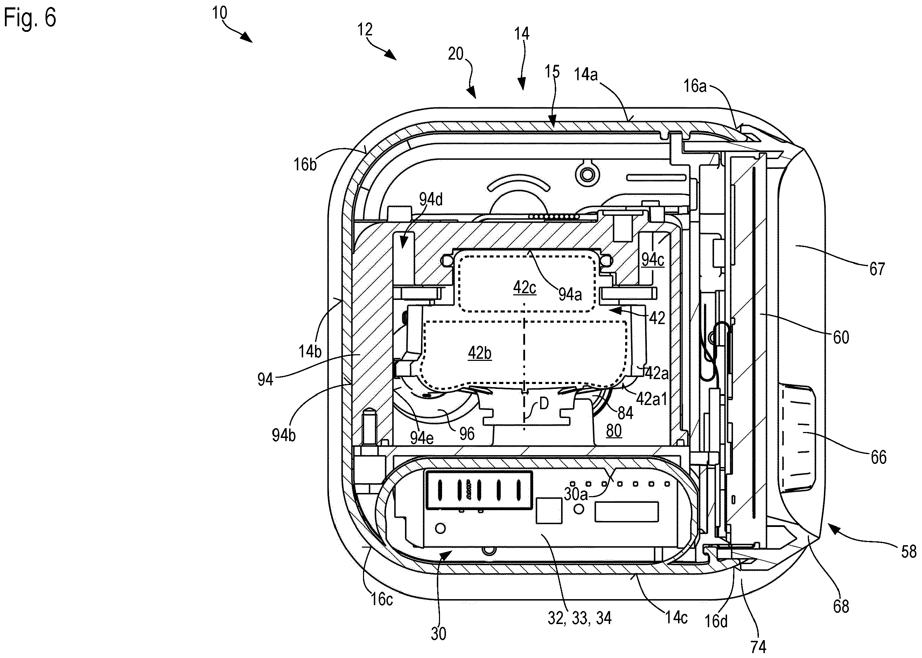

Wie in Figur 6 zu erkennen ist, ist in einem unteren Abschnitt des Gebläsegehäuses 42a ein Luftförderer 42b um eine zum ebenen Flächenanteil 14c orthogonale und zur Zeichenebene der Figuren 2 und 6 parallele Drehachse D drehbar aufgenommen. Ein über dem Luftförderer 42b gelegener elektrischer Antrieb 42c treibt den beispielhaft als Flügelrad ausgebildeten Luftförderer 42b zur Drehung an. Der untere Abschnitt des Gebläsegehäuses 42a kann als gesondertes Fördergehäuseteil ausgebildet sein, aus Kostengründen etwa aus Kunststoff. Das Fördergehäuseteil kann zur Erleichterung der Montage selbst wiederum mehrteilig ausgestaltet sein.

Ein den Antrieb 42c umgebender Teil des Gebläsegehäuses 42a ist in einer von einer Gebläse-Verbindungsfläche 94a mit geringem Spaltmaß von weniger als 1 mm, vorzugsweise von weniger als 0,3 mm, besonders bevorzugt spaltfrei, begrenzten Ausnehmung am Wärmeleitkörper 94 befestigt, beispielsweise durch Kleben oder Löten oder Schweißen oder durch Verbindungsmittel wie Schrauben. Dieser Teil des Gebläsegehäuses 42a kann als gesondertes Antriebsgehäusebauteil ausgebildet sein, zur besseren Wärmeleitung etwa aus einer Aluminium- oder Metalllegierung.

Das bevorzugt aus Aluminium im Druckgussverfahren oder durch spanende Bearbeitung aus dem Vollen hergestellte Gebläsegehäuse 42a überträgt Wärme vom Gebläse 42 zum Wärmeleitkörper 94. Da der Antrieb 42c im Betrieb des Notfall-Beatmungsgeräts 10 innerhalb des Gebläses 42 die bedeutendste Wärmequelle darstellt, umgibt die Gebläse-Verbindungsfläche 94a bevorzugt den den Antrieb 42c einhausenden Bereich des Gebläsegehäuses 42a.

Der ebenfalls bevorzugt aus Aluminium hergestellte Wärmeleitkörper 94 weist mit Abstand von der Gebläse-Verbindungsfläche 94a eine Gehäuse-Verbindungsfläche 94b auf, mit welcher der Wärmeleitkörper 94 vollflächig an der Innenseite des den ebenen Flächenanteil 14b aufweisenden Gehäuseabschnitts anliegend mit dem Gehäuse 12 verbunden ist. Bevorzugt ist der Wärmeleitkörper 94 von außen durch Durchgangslöcher in dem betreffenden Gehäuseabschnitt mit nicht gezeigten Schrauben befestigt. Die Schrauben durchsetzen die Durchgangslöcher und sind in Innengewinde am Wärmeleitkörper 94 eingedreht. So kann die Gehäuse-Verbindungsfläche 94b vollflächig spaltfrei mit dem Gehäuseabschnitt verbunden sein.

Alternativ zur Darstellung in den Figuren 2 und 6 können zwischen der Gebläse-Verbindungsfläche 94a und dem Gebläsegehäuse 42a oder/und zwischen der Gehäuse-Verbindungsfläche 94b und dem Gehäuse 12 die Wärmeleitung erhöhende Zwischenschichten angeordnet sein, beispielsweise als pastöse Schicht einer Wärmeleitpaste oder demgegenüber bevorzugt als Festkörperschicht in Gestalt einer Wärmeleitmatte.

Vom Gebläse 42 an den Wärmeleitkörper 94 übertragene Wärme folgt dem sich im Betrieb ausbildenden Temperaturgradienten am ebenen Flächenanteil 14b, wo in der Regel an der Kontaktfläche zur Außenumgebung U die niedrigste Temperatur im Pfad vom Gebläse 42 über den Wärmeleitkörper 94 bis zum Gehäuse 12 vorliegt. Am Flächenanteil 14b wird die vom Wärmeleitkörper 94 vom Gebläse 42 zum Gehäuse 12 geleitete Wärme an die Außenumgebung U durch Konvektion und Strahlung abgegeben. Eine Konvektionsströmung kann sich aufgrund des Temperaturunterschieds zwischen dem Flächenanteil 14b und der Außenumgebung U natürlich ausbilden und wird umso ausgeprägter sein, je größer der Temperaturunterschied zwischen dem Flächenanteil 14b und der Außentemperatur U ist. Da das die Mantelfläche 14 aufweisende rohrförmige Gehäusebauteil 15 bevorzugt aus dem gut wärmeleitenden Werkstoff Aluminium hergestellt ist, leitet das Gehäusebauteil 15 Wärme von dem Flächenanteil 14b auch zu benachbarten Flächenanteilen 14a, 16b, 16c, 14c, usw., so dass auch solche Flächenanteile zur Abführung von Wärme an die Außenumgebung U beitragen können, die nicht unmittelbar in Berührkontakt mit dem Wärmeleitkörper 94 stehen.

Die Gehäuse-Verbindungsfläche 94b ist mehr als doppelt so groß wie die Gebläse-Verbindungsfläche 94a.

Wie in Figur 6 zu erkennen ist, ragt ein Großteil der Außenfläche 42a1 des Gebläsegehäuses 42a in die Mischkammer 80, wo der einragende Teil der Außenfläche 42a1 von Beatmungsgas in der Mischkammer 80 benetzbar ist. So kann auch das vom Gebläse 42 geförderte Beatmungsgas zur konvektiven Kühlung des Gebläses 42 und insgesamt des Notfall-Beatmungsgeräts 10 beitragen. Die Außenfläche 42a1 umgibt die Drehachse D des Luftförderers 42b in Umfangsrichtung vollständig.

Bevorzugt ist die Kühlwirkung des Beatmungsgases und des Wärmeleitkörpers 94 so gut, dass das Notfall-Beatmungsgerät 10 keinen dedizierten Kühlerlüfter aufweist, sodass bevorzugt das Gebläse 42 zur Förderung von Beatmungsgas das einzige Gebläse im Notfall-Beatmungsgerät 10 ist.

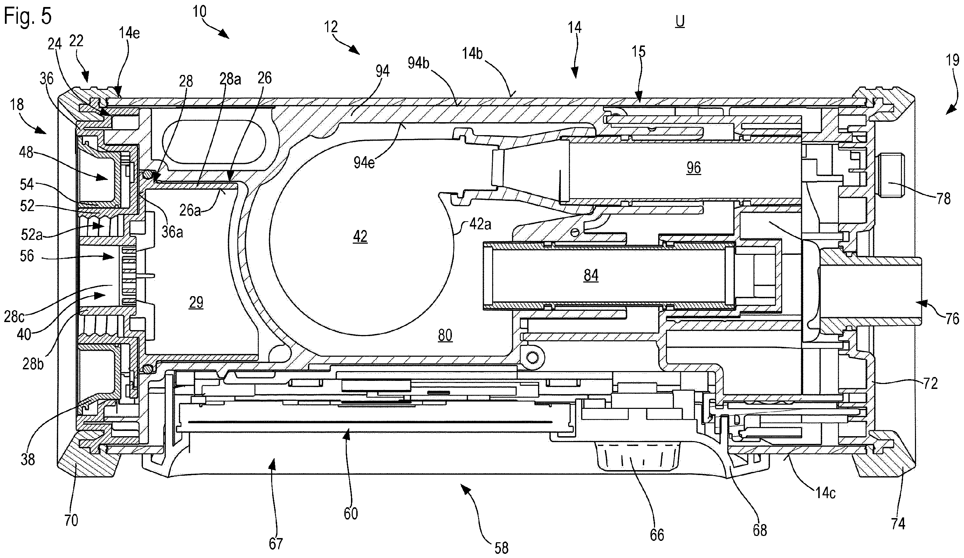

In Figur 5 ist ein Beatmungsgaskanal 96 als Ausgangskanal des Gebläses 42 zu erkennen. Auf der Druckseite des Gebläses 42 fördert das Gebläse 42 Beatmungsgas durch den Beatmungsgaskanal 96 in Richtung zur Beatmungsgas-Ausgabeöffnung 76. Der Beatmungsgaskanal 96 verläuft im dargestellten Ausführungsbeispiel platzsparend parallel zur Sondergas-Zuführleitung 84.

Im Wärmeleitkörper 94 können die Oberfläche des Wärmeleitkörpers 94 erhöhende Kanäle 94c und 94d ausgebildet sein, welche, angetrieben durch das Gebläse 42, wenigstens abschnittsweise von Beatmungsgas in der Mischkammer 80 durchströmt werden können und so zusätzlich Wärme vom Wärmeleitkörper 94 konvektiv abtransportieren. Dies erhöht die Kühlwirkung des Beatmungsgases und des Wärmeleitkörpers 94 zusätzlich.

Eine Oberfläche 94e des Wärmeleitkörpers begrenzt die Mischkammer 80 und ist von Beatmungsgas benetzbar.

Wie in Zusammenschau vor allem der Figuren 5 und 6 zu erkennen ist, umgibt der einstückige Wärmeleitkörper 94 die Mischkammer 80 von fünf Seiten. In der Mischkammer 80 sind der Luftförderer 42b und der den Luftförderer 42b umgebende Teil des Gebläsegehäuses 42a angeordnet. Der in die Mischkammer 80 einragende Teil des Gebläses 42 ist allseits mit Abstand vom Wärmeleitkörper 94 angeordnet, um eine möglichst große Fläche zu erzielen, welche Wärme an das Beatmungsgas in der Mischkammer 80 abgeben kann.

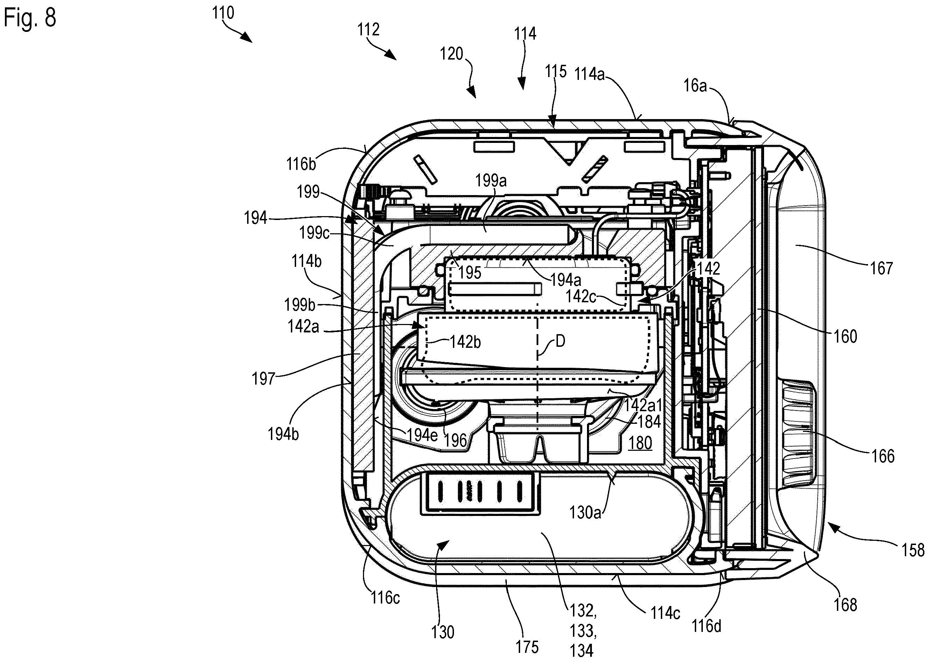

In Figur 8 ist eine zweite Ausführungsform eines erfindungsgemäßen Notfall-Beatmungsgeräts 110 in einer Querschnittsansicht dargestellt. Gleiche und funktionsgleiche Bauteile wie in der ersten Ausführungsform haben in der zweiten Ausführungsform gleiche Bezugszeichen, jedoch erhöht um die Zahl 100.

Die zweite Ausführungsform wird nachfolgend nur insofern beschrieben werden, als sie sich von der ersten Ausführungsform unterscheidet, auf deren Beschreibung ansonsten auch zur Erläuterung der zweiten Ausführungsform verwiesen wird.

Ein weniger bedeutendes Detail eines Unterschieds zwischen erster und zweiter Ausführungsform vom liegt in dem nicht mehr umlaufenden Elastomerring am Längsende des Gehäuses 112 des Beatmungsgeräts 110. Stattdessen ist an dem Gehäuse 112 an dem hinter der Schnittebene gelegenen Längsende nur ein Elastomerfuß 175 vorgesehen. Abweichend hiervon kann selbstverständlich auch die zweite Ausführungsform des Beatmungsgeräts 110 an einem oder an beiden Längsenden einen umlaufenden Elastomerring wie in der ersten Ausführungsform aufweisen.

Ein wesentlicher Unterschied liegt darin, dass der Wärmeleitkörper 194 mehrteilig ausgebildet ist. Er weist ein gebläsegehäuseseitiges Körperteil 195 und ein gesondert vom gebläsegehäuseseitigen Körperteil ausgebildetes gehäuseseitiges Körperteil 197 auf. Das gebläsegehäuseseitige Körperteil 195 weist, wie der einstückige Wärmeleitkörper 94 der ersten Ausführungsform, die Gebläse-Verbindungsfläche 194a auf. Das gehäuseseitige Körperteil 197 weist, ebenfalls wie der einstückige Wärmeleitkörper 94 der ersten Ausführungsform, die Gehäuse-Verbindungsfläche 194b auf.

Die beiden Körperteile 195 und 197 des Wärmeleitkörpers 194 sind durch wenigstens eine Heatpipe 199, im vorliegenden Beispiel durch vier hinter einander angeordnete Heatpipes, Wärme übertragend miteinander verbunden. Die dem Betrachter nächstgelegene Heatpipe 199 verdeckt dabei die dahinter gelegenen Heatpipes.

Die gleichartig gestalteten Heatpipes 199 weisen eine Wärmeaufnahmezone 199a auf, welche durch einen geradlinigen endseitigen Abschnitt der Heatpipe 199 gebildet ist. Die Wärmeaufnahmezone 199a ist zur Bereitstellung einer möglichst guten Wärmeübertragung vom gebläsegehäuseseitigen Körperteil 195 zur Wärmeaufnahmezone 199a mit dieser durch eine Lötverbindung verbunden.

An dem der Wärmeaufnahmezone 199a entgegengesetzten Endabschnitt weisen die Heatpipes 199 einen ebenfalls geradlinigen Abschnitt auf, welche eine Wärmeabgabezone 199b der Heatpipe 199 bildet. Die Wärmeabgabezone 199b ist ebenfalls zur Bereitstellung einer möglichst guten Wärmeübertragung durch eine Lötverbindung mit dem gehäuseseitigen Körperteil 197 des Wärmeleitkörpers 194 verbunden.

Im Übrigen gilt das oben zur ersten Ausführungsform Gesagte: vom Gebläse 142 wird über das Gebläsegehäuse 142a durch die Gebläse-Verbindungsfläche 194a Wärme in das gebläsegehäuseseitige Körperteil 195 übertragen. Das gebläsegehäuseseitige Körperteil 195 leitet die an es übertragene Wärme zur Wärmeaufnahmezone 199a der Heatpipe 199, welche die Wärme über eine zwischen der Wärmeaufnahmezone 199a und der Wärmeabgabezone 199b gelegene gekrümmte Zone 199c zur Wärmeabgabezone 199b leitet. Die Wärmeabgabezone 199b gibt die Wärme an das gehäuseseitige Körperteil 197 ab, welches die Wärme wiederum über das Gehäuse 112 und insbesondere über dessen dem gehäuseseitigen Körperteil 197 am nächsten gelegenen Flächenbereich 114b an die Umgebung abgibt.

Die Wärmeaufnahmezone 199a und die Wärmeabgabezone 199b schließen im dargestellten Beispiel einen Winkel von 90° ein. Die gekrümmte Zone 199c ist daher bevorzugt ein Viertel-Torus.

Im Gegensatz zur ersten Ausführungsform ist die nach innengewandte Fläche 194e des gehäuseseitigen Körperteils 197 nicht mehr in unmittelbaren Kontakt mit dem Beatmungsgas, sondern ist von diesem durch eine innere Wand des Gehäuses 112 abgeschirmt. Auch dies ist lediglich eine Konstruktionsvariation. Abweichend von der Darstellung in Figur 8 kann auch die innenliegende Fläche 194e des gehäuseseitigen Körperteils 197 die Mischkammer 180 begrenzen und vom Beatmungsgas benetzt sein. Allerdings ist es bevorzugt, wenn das Beatmungsgas nicht unmittelbar an der in der Regel erwärmten Wärmeabgabezone 199b vorbeiströmt und von dieser Wärme aufnimmt.

## Patentansprüche

1. Notfall-Beatmungsgerät (10) zur notfallmedizinischen künstlichen Beatmung von Patienten, umfassend
- ein Gehäuse (12) mit einer Umgebungsluft-Ansaugöffnung (40) und einer Beatmungsgas-Ausgabeöffnung (76), und
- ein Gebläse (42), welches dazu ausgebildet und im Gehäuse (12) angeordnet ist, um Umgebungsluft von der Umgebungsluft-Ansaugöffnung (40) zur Beatmungsgas-Ausgabeöffnung (76) zu fördern,
wobei das Gebläse (42) ein Gebläsegehäuse (42a) mit einem zum Gebläsegehäuse (42a) beweglichen Luftförderer (42b) umfasst und wobei das Gebläsegehäuse (42a) im Gehäuse (12) gehäusefest angeordnet ist,
wobei das Gebläsegehäuse (42a) unter Zwischenanordnung eines Wärmeleitkörpers (94) an dem Gehäuse (12) festgelegt ist, wobei der Wärmeleitkörper (94) ein Material umfasst oder aus einem Material gebildet ist, welches ausgewählt ist aus Leichtmetall, Buntmetall oder/und einem mit wärmeleitfähigem Füllmaterial gefüllten Kunststoff,
**dadurch gekennzeichnet, dass** eine Außenfläche (42a1) des Gebläsegehäuses (42a) in einem Abschnitt eines Strömungswegs von der Umgebungsluft-Ansaugöffnung (40) zur Beatmungsgas-Ausgabeöffnung (76) derart freiliegt, dass sie im Beatmungsbetrieb des Notfall-Beatmungsgeräts (10) von von dem Gebläse (42) geförderten Beatmungsgas benetzbar ist, wobei eine Oberfläche (94e) des Wärmeleitkörpers (94) eine Wandung eines ein Beatmungsgas umschließenden Bereichs bildet, wobei der Wärmeleitkörper (94) einen Abschnitt des Strömungswegs des Beatmungsgases von wenigstens drei Seiten unter Bildung einer Ausnehmung umgibt, wobei die von Beatmungsgas benetzbare Außenfläche (42a1) des Gebläsegehäuses (42a) in der vom Wärmeleitkörper (94) gebildeten Ausnehmung angeordnet ist.

2. Notfall-Beatmungsgerät (10) nach Anspruch 1,
**dadurch gekennzeichnet, dass** das Gebläsegehäuse (42a) starr mit dem Wärmeleitkörper (94) verbunden ist.

3. Notfall-Beatmungsgerät (10) nach einem der vorhergehenden Ansprüche, **dadurch gekennzeichnet, dass** der Wärmeleitkörper (94) starr mit dem Gehäuse (12) verbunden ist.

4. Notfall-Beatmungsgerät (10) nach einem der vorhergehenden Ansprüche, **dadurch gekennzeichnet, dass** der Wärmeleitkörper (94) wenigstens einen im Wärmeleitkörper (94) geführten Kanal (90c, 90d) aufweist, welcher wenigstens einen Teil eines Strömungswegs von der Umgebungsluft-Ansaugöffnung (40) zur Beatmungsgas-Ausgabeöffnung (76) derart bildet, dass der Kanal (90c, 90d) im Beatmungsbetrieb des Notfall-Beatmungsgeräts (10) von von dem Gebläse (42) geförderten Beatmungsgas durchströmbar ist.

5. Notfall-Beatmungsgerät (10) nach einem der vorhergehenden Ansprüche, **dadurch gekennzeichnet, dass** ein einen Antrieb (42c) einhausender Abschnitt des Gebläsegehäuses (42a) mit dem Wärmeleitkörper (94) verbunden ist und ein den Luftförderer (42b) aufweisender Abschnitt des Gebläsegehäuses (42a) in den Strömungsweg des Beatmungsgases von der Umgebungsluft-Ansaugöffnung (40) zur Beatmungsgas-Ausgabeöffnung (76) einragt.

6. Notfall-Beatmungsgerät (10) nach einem der vorhergehenden Ansprüche, **dadurch gekennzeichnet, dass** der Wärmeleitkörper (94) eine Mischkammer (80) als den Abschnitt des Strömungswegs des Beatmungsgases vorzugsweise von vier Seiten unter Bildung einer Ausnehmung umgibt, wobei die von Beatmungsgas benetzbare Außenfläche (42a1) des Gebläsegehäuses (42a) in der vom Wärmeleitkörper (94) gebildeten Ausnehmung angeordnet ist.

7. Notfall-Beatmungsgerät (10) nach einem der vorhergehenden Ansprüche, **dadurch gekennzeichnet, dass** der Wärmeleitkörper (94) den Abschnitt des Strömungswegs des Beatmungsgases von vier Seiten unter Bildung einer Ausnehmung umgibt.

8. Notfall-Beatmungsgerät (10) nach einem der vorhergehenden Ansprüche, **dadurch gekennzeichnet, dass** der Luftförderer (42b) ein relativ zum Gebläsegehäuse (42a) rotatorisch beweglicher Luftförderer (42b) ist, wobei zumindest die von Beatmungsgas benetzbare Außenfläche (42a1) des Gebläsegehäuses (42a) eine eine Drehachse (D) des Luftförderers (42b) umgebende, bevorzugt vollständig längs einer geschlossenen Bahn umgebende, Außenfläche (42a1) ist.

9. Notfall-Beatmungsgerät (10) nach einem der vorhergehenden Ansprüche, **dadurch gekennzeichnet, dass** das Gebläsegehäuse (42a) ein Material umfasst oder aus einem Material gebildet ist, welches ausgewählt ist aus Leichtmetall, Buntmetall oder/und einem mit wärmeleitfähigem Füllmaterial gefüllten Kunststoff.

10. Notfall-Beatmungsgerät (10) nach einem der vorhergehenden Ansprüche, **dadurch gekennzeichnet, dass** das Gehäuse (12) ein Material umfasst oder aus einem Material gebildet ist, welches ausgewählt ist aus Leichtmetall, Buntmetall oder/und einem mit wärmeleitfähigem Füllmaterial gefüllten Kunststoff.

11. Notfall-Beatmungsgerät (10) nach einem der vorhergehenden Ansprüche, **dadurch gekennzeichnet, dass** das Leichtmetall Aluminium, Magnesium, eine Aluminiumlegierung oder/und eine Magnesiumlegierung umfasst, dass das Buntmetall Kupfer oder/und eine Kupferlegierung umfasst, und dass das wärmeleitfähige Füllmaterial ein Metall oder/und eine Keramik oder/und Graphit oder/und Kohlenstoff, insbesondere Kohlenstoff-Nanoröhrchen, mit einer Wärmeleitfähigkeit vom mindestens 14 W/(mK) umfasst.

12. Notfall-Beatmungsgerät (10) nach einem der vorhergehenden Ansprüche, **dadurch gekennzeichnet, dass** der Wärmeleitkörper (94) eine Gebläse-Verbindungsfläche (94a) aufweist, welche einem Abschnitt des Gebläsegehäuses (42a) gegenüberliegt, und eine Gehäuse-Verbindungsfläche (94b) aufweist, welche zu einer Innenfläche des Gehäuses (12) hinweist, wobei die Gehäuse-Verbindungsfläche (94b) wenigstens doppelt so groß, vorzugsweise wenigstens viermal so groß, besonders bevorzugt wenigstens sechsmal so groß ist wie die Gebläse-Verbindungsfläche (94a).

13. Notfall-Beatmungsgerät (10) nach Anspruch 12,
**dadurch gekennzeichnet, dass** die Gehäuse-Verbindungsfläche (94b) eben ist.

14. Notfall-Beatmungsgerät (10) nach einem der vorhergehenden Ansprüche, **dadurch gekennzeichnet, dass** es eine Steuervorrichtung aufweist, welche dazu ausgebildet ist, das Gebläse (42) nur während Inspirationsphasen zu betreiben.

## Claims

1. Emergency ventilator (10) for emergency medicine artificial respiration of patients, comprising
- A housing (12) with an ambient air aspiration aperture (40) and a respiratory gas output aperture (76), and
- A fan (42) which is configured and arranged in the housing (12) in order to convey ambient air from the ambient air aspiration aperture (40) to the respiratory gas output aperture (76),
Wherein the fan (42) comprises a fan housing (42a) with an air conveyor (42b) movable relative to the fan housing (42a) and wherein the fan housing (42a) is arranged housing-tight in the housing (12),
wherein the fan housing (42a) is fixed to the housing (12) with the interposition of a heat-conducting body (94), wherein the heat-conducting body (94) comprises a material or is made from a material which is chosen out of light metal, non-ferrous metal, and/or a synthetic filled with heat-conducting filling material
**Characterized in that** an outer surface (42a1) of the fan housing (42a) is exposed in a section of a flow path from the ambient air aspiration aperture (40) to the respiratory gas output aperture (76) in such a way that during the ventilation operation of the emergency ventilator (10) it can be bathed by respiratory gas conveyed by the fan (42), wherein a surface (94e) of the heat-conducting body (94) forms a wall of a region enclosing a respiratory gas, wherein the heat-conducting body (94) surrounds a section of the flow path of the respiratory gas on at least three sides forming a recess, wherein the outer surface (42a1) of the fan housing (42a) which can be bathed by respiratory gas is arranged in the recess formed by the heat-conducting body (94).

2. Emergency ventilator (10) according to Claim 1,
**Characterized in that** the fan housing (42a) is connected rigidly with the heat-conducting body (94).

3. Emergency ventilator (10) according to one of the preceding Claims,
**Characterized in that** the heat-conducting body (94) is connected rigidly with the housing (12).

4. Emergency ventilator (10) according to one of the preceding Claims,
**Characterized in that** the heat-conducting body (94) exhibits at least one duct (90c, 90d) routed in the heat-conducting body (94) which forms at least one part of a flow path from the ambient air aspiration aperture (40) to the respiratory gas output aperture (76) in such a way that during the ventilation operation of the emergency ventilator (10) flow through the duct (90c, 90d) of respiratory gas conveyed by the fan (42) is possible.

5. Emergency ventilator (10) according to one of the preceding Claims,
**Characterized in that** a section of the fan housing (42a) which houses a drive (42c) is connected with the heat-conducting body (94) and a section of the fan housing (42a) which exhibits the air conveyor (42b) protrudes into the flow path of the respiratory gas from the ambient air aspiration aperture (40) to the respiratory gas output aperture (76).

6. Emergency ventilator (10) according to one of the preceding Claims,
**Characterized in that** the heat-conducting body (94) surrounds a mixing chamber (80) as the section of the flow path of the respiratory gas preferably on four sides forming a recess, wherein the outer surface (42a1) of the fan housing (42a) which can be bathed by respiratory gas is arranged in the recess formed by the heat-conducting body (94).

7. Emergency ventilator (10) according to one of the preceding Claims,
**Characterized in that** the heat-conducting body (94) surrounds the section of the flow path of the respiratory gas on four sides forming a recess.

8. Emergency ventilator (10) according to one of the preceding Claims,
**Characterized in that** the air conveyor (42b) is an air conveyor (42b) rotationally movable relative to the fan housing (42a), wherein at least the outer surface (42a1) of the fan housing (42a) which can be bathed by respiratory gas is an outer surface (42a1) surrounding a rotational axis (D) of the air conveyor (42b), preferably surrounding completely along a closed path.

9. Emergency ventilator (10) according to one of the preceding Claims,
**Characterized in that** the fan housing (42a) comprises a material or is made from a material which is chosen out of light metal, non-ferrous metal, and/or a synthetic filled with heat-conducting filling material.

10. Emergency ventilator (10) according to one of the preceding Claims,
**Characterized in that** the housing (12) comprises a material or is made from a material which is chosen out of light metal, non-ferrous metal, and/or a synthetic filled with heat-conducting filling material.

11. Emergency ventilator (10) according to one of the preceding Claims,
**Characterized in that** the light metal comprises aluminum, magnesium, an aluminum alloy, and/or a magnesium alloy, that the non-ferrous metal comprises copper and/or a copper alloy, and that the heat-conducting filling material comprises a metal, and/or a ceramic, and/or graphite, and/or carbon, in particular carbon nanotubes, with a heat conductance of at least 14 W/(mK).

12. Emergency ventilator (10) according to one of the preceding Claims,
**Characterized in that** the heat-conducting body (94) exhibits a fan joint-face (94a) which lies opposite a section of the fan housing (42a) and exhibits a housing joint-face (94b) which faces towards an inner surface of the housing (12), wherein the housing joint-face (94b) is at least twice as large, preferably at least four times as large, especially preferably at least six times as large as the fan joint-face (94a).

13. Emergency ventilator (10) according to Claim 12,
**Characterized in that** the housing joint-face (94b) is planar.

14. Emergency ventilator (10) according to one of the preceding Claims,
**Characterized in that** it exhibits a control device which is configured to operate the fan (42) only during inspiratory phases.

## Revendications

1. Respirateur d'urgence (10) pour la respiration artificielle de patients en médecine d'urgence, comprenant
- un boîtier (12) avec une ouverture d'aspiration d'air ambiant (40) et une ouverture de distribution de gaz respiratoire (76), et
- une soufflante (42) qui est conçue et disposée dans le boîtier (12) pour transporter l'air ambiant de l'ouverture d'aspiration d'air ambiant (40) à l'ouverture de distribution de gaz respiratoire (76),
dans lequel la soufflante (42) comprend un boîtier de soufflante (42a) avec un transporteur d'air (42b) mobile par rapport au boîtier de soufflante (42a) et dans lequel le boîtier de soufflante (42a) est disposé de manière fixe dans le boîtier (12), dans lequel le boîtier de soufflante (42a) est fixé sur le boîtier (12) avec interposition d'un corps conducteur de chaleur (94), dans lequel le corps conducteur de chaleur (94) comprend un matériau ou est formé d'un matériau qui est choisi parmi un métal léger, un métal non ferreux ou/et une matière plastique remplie d'un matériau de remplissage conducteur de chaleur,
**caractérisé en ce qu'**une surface extérieure (42a1) du boîtier de soufflante (42a) est exposée dans une section d'un trajet d'écoulement depuis l'ouverture d'aspiration d'air ambiant (40) jusqu'à l'ouverture de distribution de gaz respiratoire (76) de telle sorte qu'elle peut être mouillée par le gaz respiratoire transporté par la soufflante (42) pendant le fonctionnement du respirateur d'urgence (10), dans lequel une surface (94e) du corps conducteur de chaleur (94) forme une paroi d'une zone entourant un gaz respiratoire, dans lequel le corps conducteur de chaleur (94) entoure une section du trajet d'écoulement du gaz respiratoire d'au moins trois côtés en formant un évidement, dans lequel la surface extérieure (42a1) du boîtier de soufflante (42a) pouvant être mouillée par le gaz respiratoire est disposée dans l'évidement formé par le corps conducteur de chaleur (94).

2. Respirateur d'urgence (10) selon la revendication 1,
**caractérisé en ce que** le boîtier de soufflante (42a) est relié de manière rigide au corps conducteur de chaleur (94).

3. Respirateur d'urgence (10) selon l'une des revendications précédentes, **caractérisé en ce que** le corps conducteur de chaleur (94) est relié rigidement au boîtier (12).

4. Respirateur d'urgence (10) selon l'une des revendications précédentes, **caractérisé en ce que** le corps conducteur de chaleur (94) présente au moins un canal (90c, 90d) guidé dans le corps conducteur de chaleur (94), qui forme au moins une section d'un trajet d'écoulement depuis l'ouverture d'aspiration d'air ambiant (40) jusqu'à l'ouverture de distribution de gaz respiratoire (76) de telle sorte que le canal (90c, 90d) peut être traversé par le gaz respiratoire transporté par la soufflante (42) pendant le fonctionnement du respirateur d'urgence (10).

5. Respirateur d'urgence (10) selon l'une des revendications précédentes, **caractérisé en ce qu'**une partie du boîtier de soufflante (42a) renfermant un entraînement (42c) est reliée au corps conducteur de chaleur (94) et une partie du boîtier de soufflante (42a) présentant le transporteur d'air (42b) fait saillie dans le trajet d'écoulement du gaz respiratoire depuis l'ouverture d'aspiration d'air ambiant (40) jusqu'à l'ouverture de distribution de gaz respiratoire (76).

6. Respirateur d'urgence (10) selon l'une des revendications précédentes, **caractérisé en ce que** le corps conducteur de chaleur (94) entoure une chambre de mélange (80) comme la section du trajet d'écoulement du gaz respiratoire, de préférence de quatre côtés, en formant un évidement, dans lequel la surface extérieure (42a1) du boîtier de soufflante (42a) pouvant être mouillée par le gaz respiratoire est disposée dans l'évidement formé par le corps conducteur de chaleur (94).

7. Respirateur d'urgence (10) selon l'une des revendications précédentes, **caractérisé en ce que** le corps conducteur de chaleur (94) entoure la section du trajet d'écoulement du gaz respiratoire sur quatre côtés en formant un évidement.

8. Respirateur d'urgence (10) selon l'une des revendications précédentes, **caractérisé en ce que** le transporteur d'air (42b) est un transporteur d'air (42b) mobile en rotation par rapport au boîtier de soufflante (42a), dans lequel au moins la surface extérieure (42a1) du boîtier de soufflante (42a) pouvant être mouillée par le gaz respiratoire est une surface extérieure (42a1) entourant un axe de rotation (D) du transporteur d'air (42b), de préférence l'entourant entièrement le long d'une trajectoire fermée.

9. Respirateur d'urgence (10) selon l'une des revendications précédentes, **caractérisé en ce que** le boîtier de soufflante (42a) comprend un matériau ou est formé d'un matériau choisi parmi un métal léger, un métal non ferreux ou/et une matière plastique remplie d'un matériau de remplissage conducteur de chaleur.

10. Respirateur d'urgence (10) selon l'une des revendications précédentes, **caractérisé en ce que** le boîtier (12) comprend un matériau ou est formé d'un matériau qui est choisi parmi un métal léger, un métal non ferreux ou/et une matière plastique remplie d'un matériau de remplissage conducteur de chaleur.

11. Respirateur d'urgence (10) selon l'une des revendications précédentes, **caractérisé en ce que** le métal léger comprend de l'aluminium, du magnésium, un alliage d'aluminium ou/et un alliage de magnésium, **en ce que** le métal non ferreux comprend du cuivre ou/et un alliage de cuivre, et **en ce que** le matériau de remplissage conducteur de chaleur comprend un métal ou/et une céramique ou/et du graphite ou/et du carbone, notamment des nanotubes de carbone, ayant une conductivité de chaleur d'au moins 14 W/(mK).

12. Respirateur d'urgence (10) selon l'une des revendications précédentes, **caractérisé en ce que** le corps conducteur de chaleur (94) comprend une surface de connexion de soufflante (94a) qui est opposée à une partie du boîtier de soufflante (42a) et comprend une surface de connexion de boîtier (94b), qui fait face à une surface intérieure du boîtier (12), dans lequel la surface de connexion de boîtier (94b) est au moins deux fois plus grande, de préférence au moins quatre fois plus grande, de manière particulièrement préférée au moins six fois plus grande que la surface de connexion de soufflante (94a).

13. Respirateur d'urgence (10) selon la revendication 12,
**caractérisé en ce que** la surface de connexion de boîtier (94b) est plane.

14. Respirateur d'urgence (10) selon l'une des revendications précédentes, **caractérisé en ce qu'**il comporte un dispositif de commande adapté pour ne faire fonctionner la soufflante (42) que pendant les phases d'inspiration.
